(19) Europäisches Patentamt
European Patent Office
Office européen des brevets

(11) Publication number: **0 184 170 B1**

# (12) EUROPEAN PATENT SPECIFICATION

(45) Date of publication of patent specification: **16.10.91**

(51) Int. Cl.5: **C07D 263/20**, A61K 31/42

(21) Application number: **85115243.9**

(22) Date of filing: **30.11.85**

The file contains technical information submitted after the application was filed and not included in this specification

## (54) Aminomethyl oxooxazolidinyl benzene derivatives useful as antibacterial agents.

(30) Priority: **05.12.84 US 676745**

(43) Date of publication of application:
**11.06.86 Bulletin 86/24**

(45) Publication of the grant of the patent:
**16.10.91 Bulletin 91/42**

(84) Designated Contracting States:
**AT BE CH DE FR GB IT LI LU NL SE**

(56) References cited:
**EP-A- 0 050 827**
**EP-A- 0 081 200**
**EP-A- 0 127 902**
**FR-A- 2 500 450**
**US-A- 3 687 965**

**Chem. Soc. Rev. (1979) 18, 563-580**

(73) Proprietor: **E.I. DU PONT DE NEMOURS AND COMPANY**
**1007 Market Street**
**Wilmington Delaware 19898(US)**

(72) Inventor: **Gregory, Walter Adelman**
**104 Rockingham Drive**
**Wilmington Delaware 19803(US)**

(74) Representative: **von Kreisler, Alek, Dipl.-Chem. et al**
**Deichmannhaus am Hauptbahnhof**
**W-5000 Köln 1(DE)**

EP 0 184 170 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Technical Field

This invention relates to novel aminomethyl oxooxazolidinyl benzene derivatives; to pharmaceutical compositions containing them, and to methods of using them to alleviate bacterial infections.

Background of the Invention

At the present time, no existing antibacterial product provides all features deemed advantageous. There is continual development of resistance by bacterial strains. A reduction of allergic reactions and of irritation at the site of injection, and greater biological half-life (i.e., longer in vivo activity) are currently desirable features for antibacterial products.

U.S. Patent 4,128,654 issued to Fugitt et al. on December 5, 1978, discloses, among others, compounds of the formula:

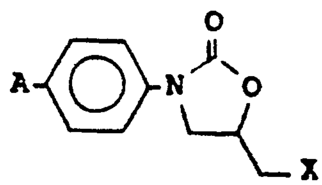

where

A = $RS(O)_n$;
X = Cl, Br or F;
R = $C_1$-$C_3$ alkyl; and
n = 0, 1 or 2.

The compounds are disclosed as being useful in controlling fungal and bacterial diseases of plants.

U.S. Reissue Patent 29,607 reissued April 11, 1978 discloses derivatives of 5-hydroxymethyl-3-substituted-2-oxazolidinones of the formula:

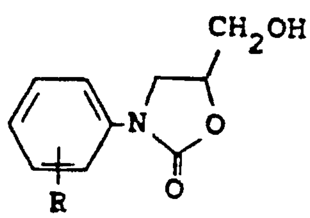

where R is H, F, $CH_3$, or $CF_3$. Such compounds are described as having antidepressive, tranquilizing, sedative, and antiinflammatory properties.

U.S. Patent 4,250,318, which was issued on February 10, 1981, discloses antidepressant compounds of the formula:

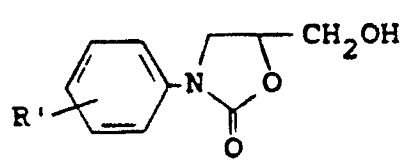

where R' can be, among others, a para-n-pentylamino group, an $SR_1$ group where $R_1$ is $C_1$-$C_5$ alkyl, or an acetylmethylthio group.

U.S. Patent 4,340,606, equivalent to EP-A-50 827, discloses antibacterial agents of the general formula:

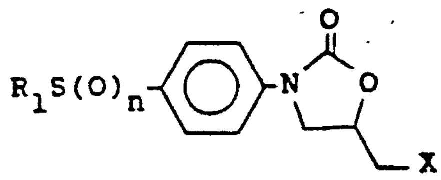

where

R1 = $CH_3$, $C_2H_5$, $CF_2H$, $CF_3$ or $CF_2CF_2H$; and

X = $OR_2$ ($R_2$ = H or various acyl moieties).

U.S. Patent 3,687,965, issued to Fauran et al. on August 29, 1972, discloses compounds of the formula:

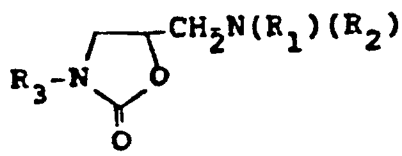

where

-N(R1)(R2) represents either dialkylamino radical in which the alkyl portions have one to five carbon atoms, or a heterocyclic amino radical which may be substituted by an alkyl radical having one to five carbon atoms or by a pyrrolidinocarbonylmethyl radical, and

R3 represents a phenyl radical which may be substituted by one or more of the following radicals:
an alkoxy radical having one to five carbon atoms;
a halogen atom;
a trifluoromethyl radical, or
a carboxyl radical which may be esterified.

The patent states that these compounds possess hypotensive, vasodilatatory, spasmolytic, sedative, myorelaxant, analgesic and antiinflammatory properties. There is no mention of antibacterial properties.

Belgian Patent 892,270, published August 25, 1982, discloses monoamine oxidase inhibitors of the formula

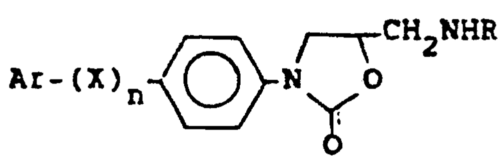

where

R is H, $C_1$-$C_4$ alkyl or propargyl;

Ar is phenyl, optionally substituted by halo or trifluoromethyl;

n is 0 or 1; and

X is $-CH_2CH_2-$, $-CH=CH-$, an acetylene group or $-CH_2O-$.

U.S. Patent 4,461,773, equivalent to EP-A-81 200, a continuation-in-part of U.S. Patent Application 417,569 filed September 15, 1982 discloses antibacterial agents of the formula

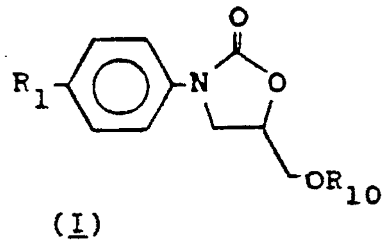

(I)

wherein, for the ℓ, and mixtures of the d and ℓ stereoisomersof the compound,

$R_1$ is $R_2SO_2$,

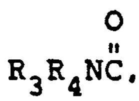 or 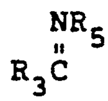 ;

$R_2$ is $-NR_3R_4$, $-N(OR_3)R_4$, $-N_3$, $-NHNH_2$, $-NX_2$, $-NR_6X$, $-NXZ$,

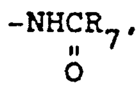 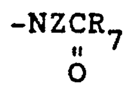

or $-N=S(O)_nR_8R_9$;

$R_3$ and $R_4$ are independently H, alkyl of 1-4 carbons or cycloalkyl of 3-8 carbons;

$R_5$ is $NR_3R_4$ or $OR_3$;

$R_6$ is alkyl of 1-4 carbons;

$R_7$ is alkyl of 1-4 carbons, optionally substituted with one or more halogens;

$R_8$ and $R_9$ are independently alkyl of 1-4 carbons or, taken together are $-(CH_2)_p-$;

$R_{10}$ is H, alkyl of 1-3 carbons,

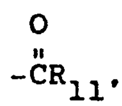

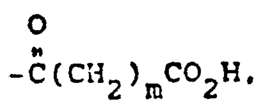 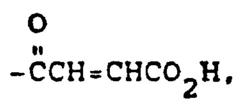 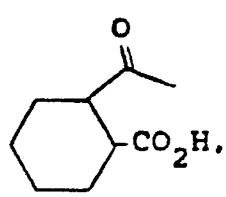

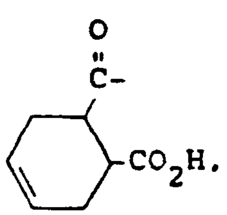 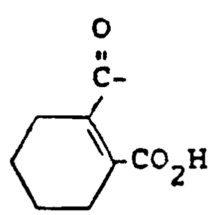 or 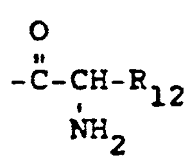 ;

$R_{11}$ is alkyl of 1-12 carbons;

$R_{12}$ is H, alkyl of 1-5 carbons, $CH_2OH$ or $CH_2SH$;

X is Cl, Br or I;

| | |
|---|---|
| Z | is a physiologically acceptable cation; |
| m | is 2 or 3; |
| n | is 0 or 1; and |
| p | is 3, 4 or 5; |

and when $R_{10}$ is alkyl of 1-3 carbons, $R_1$ can also be $CH_3S(O)_q$ where q is 0, 1 or 2; or a pharmaceutically acceptable salt thereof.

EP-A-127 902 discloses aminomethyl oxoazolidinyl benzene derivatives useful as antibacterial agents.

None of the cited references nor any known references suggest the novel antibacterial compounds of this invention.

## Summary of the Invention

The novel compounds of the instant invention possess useful antibacterial activity in both *in vitro* and *in vivo* tests. Specifically, one aspect of this invention relates to compounds having the formula:

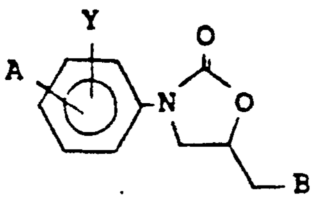

(I)

wherein, for the ℓ, and mixtures of the d and ℓ stereoisomers of the compound,

| | |
|---|---|
| A | is halogen, alkynyl of 2-5 carbon atoms, |

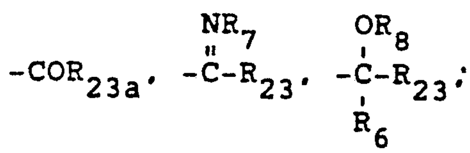

| | |
|---|---|
| $R_5$ and $R_6$ | are alkyl of 1-4 carbon atoms; |
| $R_7$ | is |

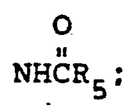

| | |
|---|---|
| $R_8$ | is H; |
| $R_{23}$ | is H; |
| $R_{23a}$ | is alkyl of 1-4 carbon atoms substituted with one or more halogens; |
| B | is |

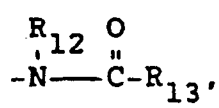

| | |
|---|---|
| $R_{12}$ | is H; |
| $R_{13}$ | is $C_1$-$C_4$ alkyl; $-OR_{18}$; |
| $R_{18}$ | is $C_1$-$C_4$ alkyl |

or a pharmaceutically suitable salt thereof; provided that: when A is F, then B is not $NHCO_2CH_3$, and when B is $-NH-CO-CH_3$, then A and Y taken together cannot be $O-CH_2-O$.

Preferred, for their high antibacterial activity or ease of synthesis, or both, are compounds of formula I where:

(1) A, substituted in the para position, is

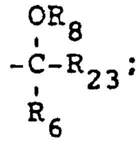

(2) B is

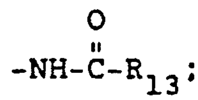

$R_{13}$ is $CH_3$, $OR_{18}$;

Preferred because of high antibacterial activity are compounds of formula I having the absolute configuration depicted:

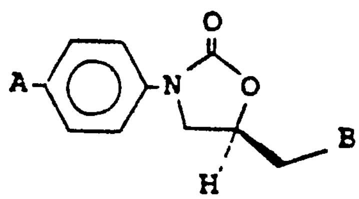

More preferred because of high antibacterial activity are compounds of formula I having the absolute configuration depicted:

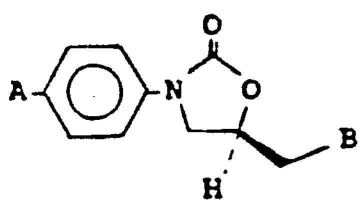

and
where A is $CH_3CH(OH)$-; and
where B is -$NHCOCH_3$,.

Specifically preferred for their high antibacterial activity is the following compound:

- (ℓ)-N-[3-[4-(1-hydroxyethyl)phenyl]-2-oxo-5-oxazolidinylmethyl]acetamide.

Another aspect of this invention relates to a pharmaceutical composition comprising a suitable pharmaceutical carrier and an antibacterially effective amount of at least one of the aforesaid compounds. Yet another aspect of the invention relates to a method for alleviating bacterial infection in a mammal which comprises administering to the mammal an antibacterially effective amount of at least one of the aforesaid compounds.

Detailed Description

The compounds of formula I, contain at least one chiral center, and as such exist as two individual isomers or as a mixture of both. This invention relates to the levorotatory isomer (ℓ), as well as mixtures containing both the d and the ℓ isomers. Additional chiral centers may be present in the groups A and/or B and this invention relates to all possible stereoisomers and various isomeric mixtures in these groups.

For the purposes of this invention, the ℓ-isomer of compounds of formula I, is intended to mean

compounds of the configuration depicted:

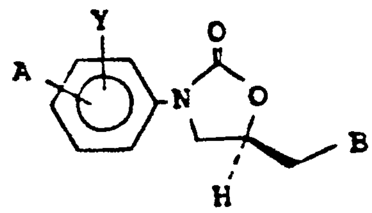

<u>Synthesis</u>

Compounds of Formula (I) can be prepared as follows:

<u>Scheme 1</u>:

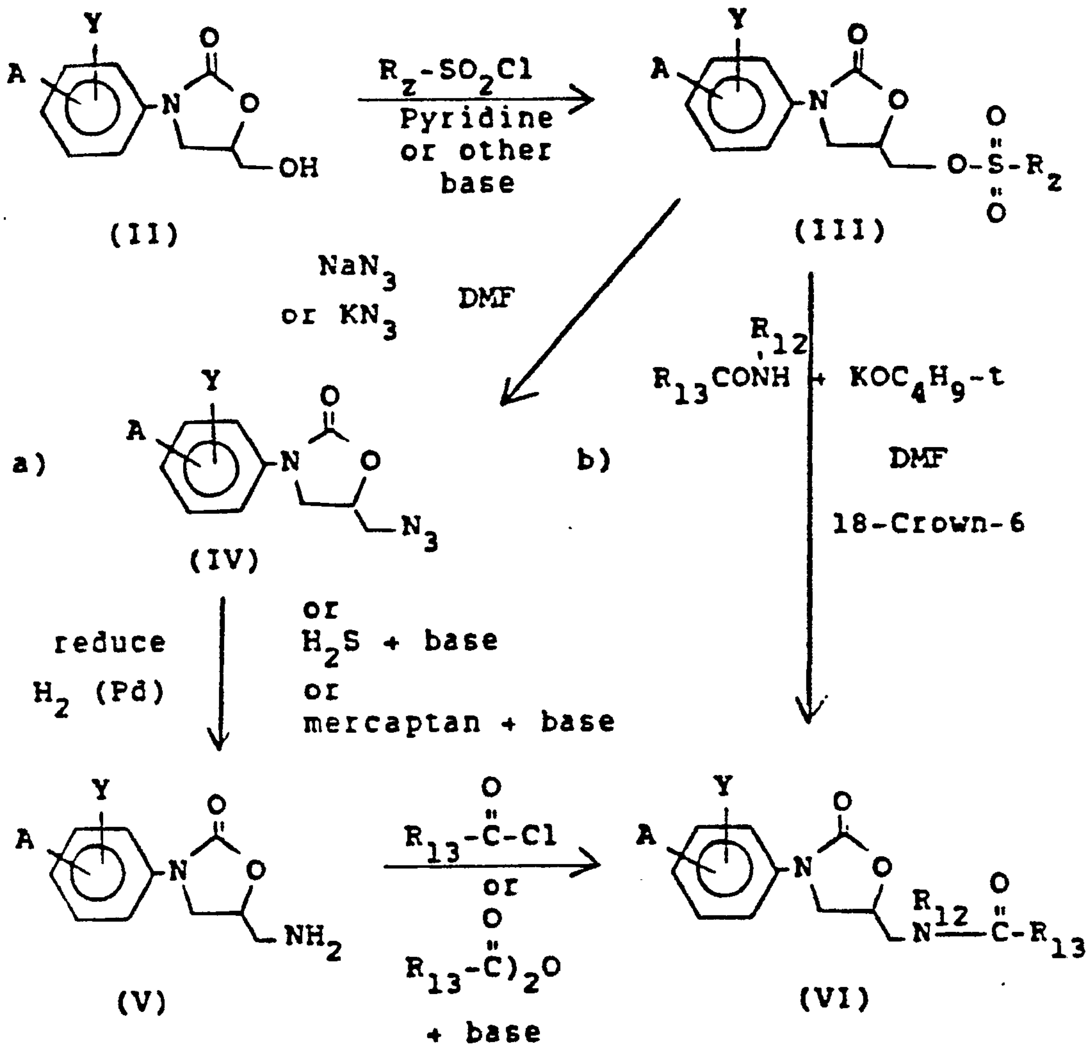


(II) (III) a) (IV) b) (V) (VI)

Where $R_2$ may be 4-tolyl, phenyl, 4-chlorophenyl, $C_1$-$C_4$ alkyl or haloalkyl, such as trifluoromethyl.
When either synthetic path a) or path b) is used, the group A may be -H or any of the groups previously shown.

Compounds of Formula (II) may be converted to sulfonate esters (III) by reaction with the appropriate sulfonyl halide or sulfonic anhydride in a solvent plus a base or in a basic organic solvent such as pyridine.

As solvents, 1,2-dimethoxyethane, dioxane, bis-(2-methoxyethyl)ether, N,N-dimethylformamide (DMF), N,N-dimethylacetamide (DMAc), acetonitrile, or tetramethylenesulfone may be used. As a base, triethylamine, N-methylmorpholine, tributylamine or one of the heterocyclic bases can be used.

Compounds (III) may be reacted with sodium, potassium, lithium, cesium or rubidium azides in a dipolar aprotic solvent such as DMF, N-methylpyrrolidone, DMAc, sulfolane, dimethylsulfoxide, tetramethyl-urea, hexamethylphosphoramide (HMPA), etc. along with the appropriate catalyst such as 18-crown-6 or 15-crown-5 for sodium and potassium azide and 12-crown-4 for lithium azide. This reaction is carried out from about 60° to 125° C, with the preferred temperatures being 70° to 90° C. The products are azides of structure (IV).

The azides (IV) may be reduced by any of several methods, including hydrogenation over palladium-on-charcoal. It is also possible to reduce the azides by treating with 1,3-propanedithiol and a base such as triethylamine. Azides may also be reduced to amines by hydrogen sulfide and by trivalent phosphorous compounds such as trimethylphosphine and trimethylphosphite, and by mercaptans such as mercaptoacetic acid. Reduction with hydrogen can be used where A is a functional group resistant to hydrogenation or hydrogenolysis. The reduction is carried out using a solvent such as ethanol, methanol, 1,2-dimethoxyethane, acetic acid, trifluoroacetic acid, or isopropanol. A solution may be stirred at ambient temperature with palladium-on-charcoal catalyst present and the hydrogen introduced at atmospheric pressure through a glass frit. In some instances the reduction is exothermic.

The reduction using 1,3-propanedithiol is carried out in methanol or other alcohol solvents containing an equivalent of triethylamine, by warming until $N_2$ evolution occurs. At ambient temperatures, slow reduction occurs. Temperatures of 20° to 100° C may be used; temperatures of 40° to 60° C are preferred. Warming an azide (IV) with trimethylphosphite causes a rapid evolution of $N_2$. The reaction may be carried out in 1,2-dimethoxyethane or bis-(2-methoxyethyl)ether and the crude intermediate, when hydrolyzed with water or acid, gives the desired amine (V).

The aminomethyl compounds (V) are acylated by reaction of the amine with an acid chloride or anhydride in a basic solvent such as pyridine or by reaction in a water miscible solvent such as THF or 1,2-dimethoxyethane in the presence of an aqueous base such as sodium hydroxide or potassium hydroxide, sodium bicarbonate or sodium carbonate. When pyridine is used as solvent for the reaction, the acid chloride or anhydride is added to the mixture at 0° to 10° C. The reaction may be carried out between -30° and 50° C. With very reactive acid chlorides or anhydrides such as trifluoromethanesulfonyl chloride or anhydride the reaction is preferably carried out at -60° to -40° C. The acylations using aqueous bases are done by stirring the amine (V) in a water miscible solvent such as tetrahydrofuran (THF), 1,2-dimethoxyethane, or dioxane and adding 1-5 N NaOH to keep the mixture basic as the acid chloride or anhydride is added, while keeping the temperature between -5° and 20° C. The compounds (V) can also be acylated by any of the standard peptide synthesis methods where the free acid is reacted with the amine using N,N-dicyclohexylcarbodiimide, or where a mixed anhydride is first formed from the acid using a chloroformate ester and a tertiary base such as triethylamine, followed by reaction with the amine. In the mixed anhydride procedure, the acid to be used is allowed to react with a chloroformate such as ethyl chloroformate or isobutyl chloroformate in a solvent such as THF, DMF or 1,2-dimethoxyethane, in the presence of a tertiary base such as triethylamine or N-methylmorpholine at -30° to 10° C. To this mixture the amine (V) is added and the mixture stirred at -10° C for 1-5 hours. When N,N-dicyclohexylcarbodiimide is used as the condensing agent, the conditions and solvents may be the same but it is often advantageous to add N-hydroxyphthalimide or N-hydroxysuccinimide.

Further, these amines may be acylated by reaction with esters such as methyl dichloroacetate, ethyl trifluoroacetate or n-butyl formate. In this method, the amine (V) is combined with the ester and a solvent such as 1,2-dimethoxyethane, bis-(2-methoxyethyl)ether or toluene (in some cases the ester may be used as the solvent) and the mixture is heated at reflux until the reaction is shown to be complete by an assay such as thin-layer chromatography. More reactive esters such as p-nitrophenyl esters, pentafluorophenyl esters, thio esters, enol esters, N-hydroxyphthalimide esters, N-hydroxysuccinimide esters, 1-hydroxybenzotriazole esters, 2,4,5-trichlorophenyl esters, and pentachlorophenyl esters, may be used. Further, other acylating agents such as acyl azides, acyl imidazoles and acyl phosphates, may be used.

When synthetic path b) is used, the sulfonate ester (III) is allowed to react with an amide in the form of its sodium or potassium salt, generated using NaH, KH or $KOC_4H_9$-t in a dipolar aprotic solvent such as DMF, DMAc, HMPA, N-methylpyrrolidinone, or tetramethylenesulfone. To the salt preparation is added the sulfonate ester (III) and the mixture is heated to 30° to 150° C. A catalyst such as 18-crown-6 may be used. Heating is continued for 3-50 hours.

In Scheme 1, the starting compound (II) may be dℓ- (the racemate) or the ℓ-isomer. The ℓ-isomer is a precursor for the preferred ℓ-amides (VI).

When the acylating group is derived from an α-amino acid and $R_{13}$ contains an amino function it is necessary to protect that amino function with one of the commonly used protective groups such as benzyloxycarbonyl, t-butyloxycarbonyl, 9-fluorenylmethyloxycarbonyl, or phthaloyl. Following the acylation, the protective group is removed by one of the standard methods to which the oxazolidinone ring is inert. The benzyloxycarbonyl group may be removed by hydrogenation in a solvent such as methanol, DMF, acetic acid, or mixtures of these solvents, using a catalyst such as 10% palladium-on-carbon or palladium black (100 to 500 mg of catalyst per mmole of compound). Alternatively the benzyloxycarbonyl group may be removed by dissolving the compound in acetic acid, adding an equal volume of 4 N HBr in acetic acid, and keeping the solution at room temperature for 1 to 5 hours. The $N^{\alpha}$-t-butyloxycarbonyl groups are removed by hydrolysis with trifluoroacetic acid at room temperature.

Scheme 2:

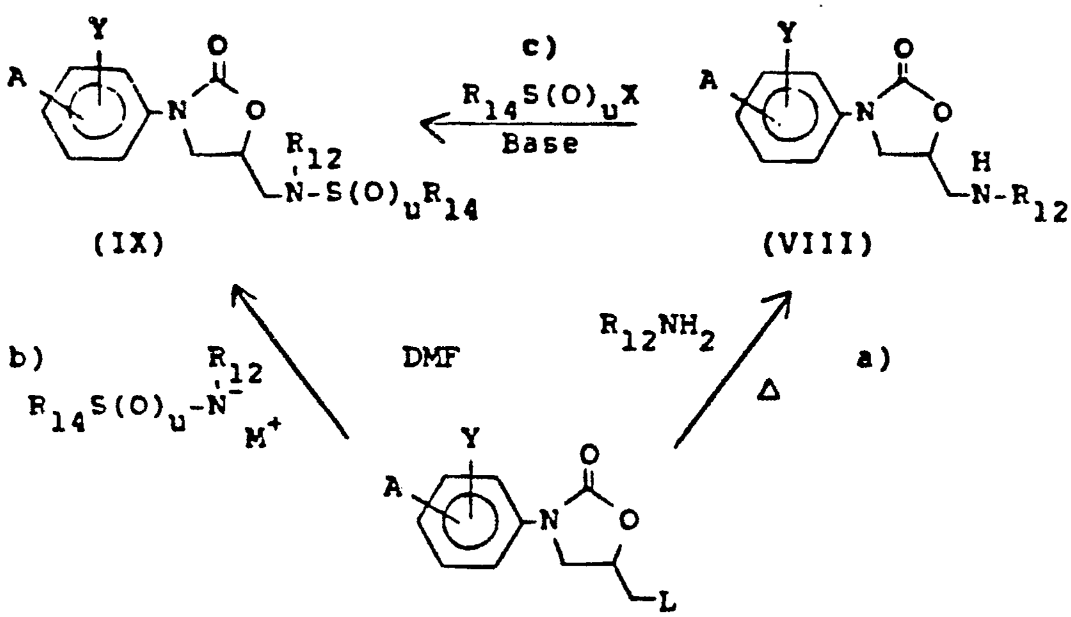

(IX)
(VIII)
(VII)

Compounds of formula (I) which may be made using the procedures of Scheme 2 are those where A is H or any of the groups previously shown. L may be any suitable leaving group such as I, Br, Cl, benzenesulfonyloxy, 4-toluenesulfonyloxy, methanesulfonyloxy or trifluoromethanesulfonyloxy. In route a) the compound (VII) is allowed to react with ammonia or an amine in a solvent such as ethanol at temperatures of 50° to 150°C. Where the amine or solvent is low-boiling, the reaction is carried out in a sealed vessel to allow the desired temperature to be reached. The solvent may be ethanol, DMF, DMAc, N-methylpyrrolidinone, tetramethylenesulfone, or HMPA. The reaction time may be 1 to 24 hours. Where (VII) is optically active (i.e., the ℓ-isomer) the product is optically active. The acylation of product VIII is carried out as described for Scheme 1, Path a).

The reaction of (VII) with the anion of a sulfonamide shown in Scheme 2, Path b) is carried out in a polar solvent such as DMF, DMAc, N-methylpyrrolidinone, tetramethylenesulfone, or HMPA. In some cases the use of a catalyst such as 18-crown-6 may improve the reaction. Temperatures of 50° to 150°C are employed; the time for the reaction can vary between 2 to 48 hours.

Alternatively, the sulfonamides (IX) can be prepared by reaction of the amine (VIII) with a sulfonyl halide in the presence of a base such as triethylamine or a basic solvent such as pyridine [Path c)].

Scheme 3:

a)

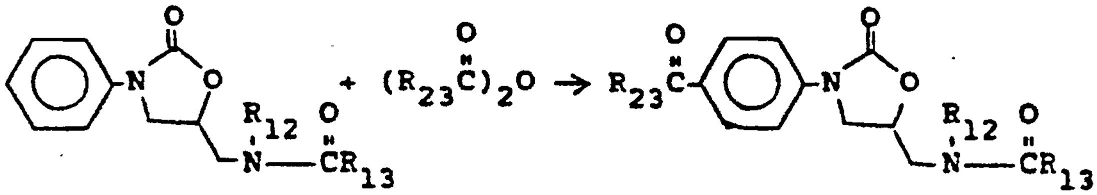

(VI: where A=H) (XVI)

b)

(XVI) 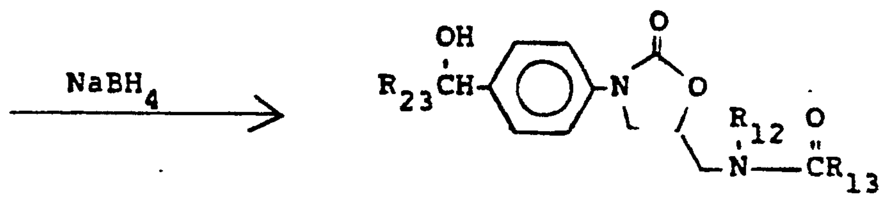


(XVII)

The acylation of Scheme 3, Path a) is carried out by reacting the compounds of formula (VI)(A = H) with an excess of an acid anhydride in methanesulfonic acid. This acylation may be carried out at room temperature, over time periods of 2-10 hours. Alternatively, the acylation can be carried out with an appropriate carboxylic acid in the presence of a mixture of phosphorous pentoxide or methanesulfonic anhydride in methanesulfonic acid. During the reaction, the amide nitrogen may be simultaneously substituted with a second acyl group which is removed in the aqueous work-up or by treatment with an alcohol such as methanol.

The acylated compounds (XVI) may be reduced to the corresponding alcohol (XVII) as shown in Scheme 3, Path b). The reduction can be carried out using sodium borohydride in an alcohol solvent such as ethanol, or via other mild metal borohydride reagents such as lithium borohydride, lithium tri-t-butoxyaluminum hydride, tetramethylammonium borohydride or the like.

The reactions of Scheme 3 may be carried out starting with the ℓ-isomer of (XVI) where A = H to give products of the preferred ℓ-form.

An alternative synthesis of the glycinamides of Formula I where B is

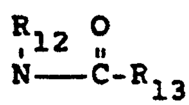

wherein $R_{13}$ is $CH_2NH_2$ as well as compounds where $R_{13}$ is $CH_2N_3$ is shown in Scheme 4.

Scheme 4:

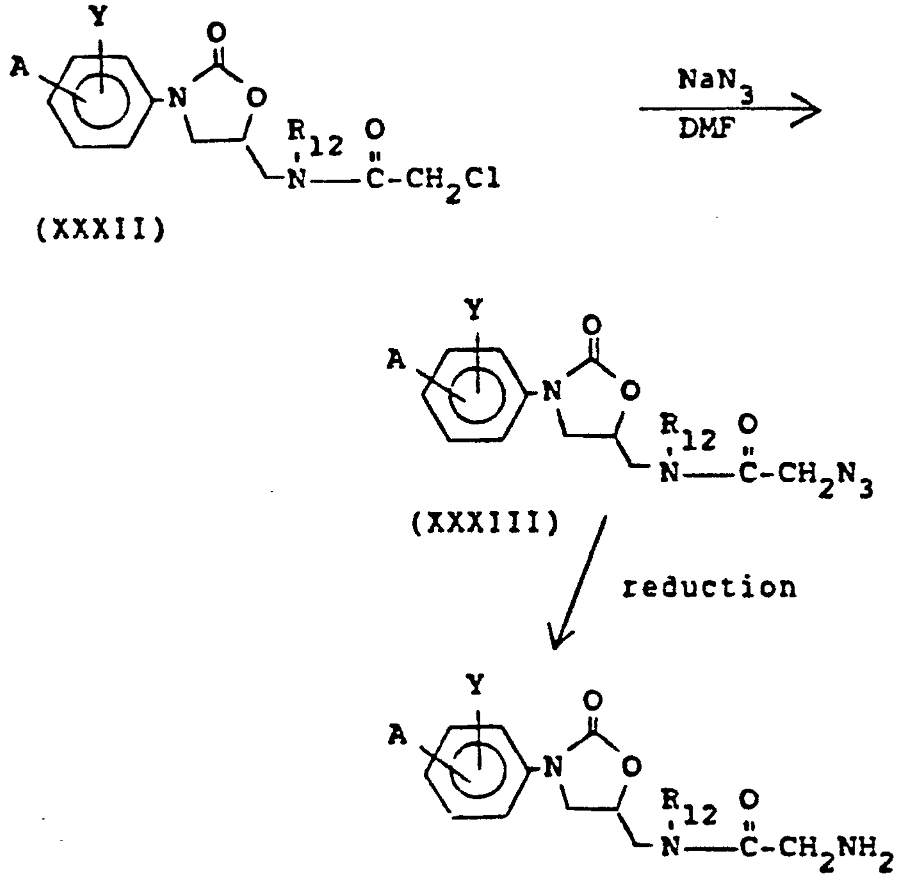


(XXXII)

(XXXIII)

(XXXIV)

Glycine amides (XXXIV) may be prepared by making the chloroacetyl or bromoacetyl or iodoacetyl compounds (XXXII) followed by reacting these with sodium azide in dimethylsulfoxide or other dipolar aprotic solvents to give the azidoacetyl compounds (XXXIII). The azidoacetyl compounds then may be reduced by hydrogen using a palladium catalyst or by any of the other reduction methods such as 1,3-propanedithiol and triethylamine, thioglycolic acid or hydrogen sulfide. The products are the glycine amides (XXXIV).

The compounds of Formula I where A is $CR_{23}(OR_{16})OR_{17}$, or

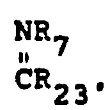

are obtained as shown in Scheme 5.

Scheme 5:

a)

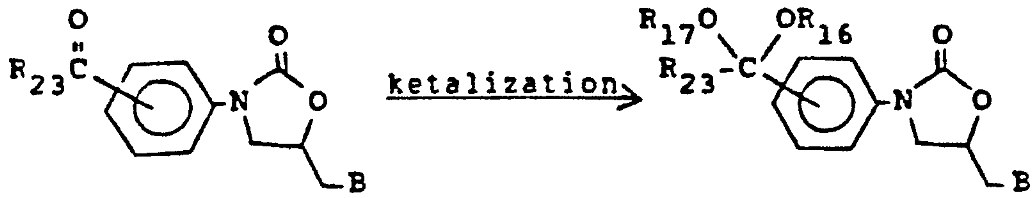


(XXXV) (XXXVI)

b)

(XXXV)

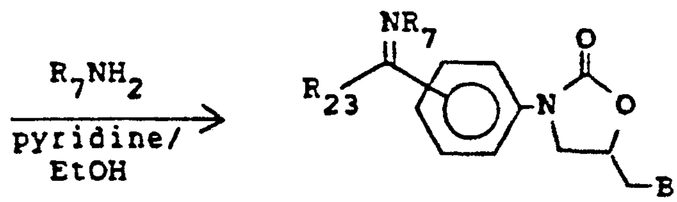


(XXXVII)

Ketalization, as shown in Scheme 5, Path a) can be carried out by reacting the ketone derivative (XXXV) with an appropriate ortho ester in an alcohol solvent such as ethanol in the presence of an acid catalyst such as p-toluenesulfonic acid, sulfuric acid, methanesulfonic acid or the like to give the corresponding dialkyl acetal. The reaction can be promoted by removing the ester by-product by distillation. Cyclic ketals can be prepared by treatment of the ketone with ethylene glycol or 1,3-propanediol in an inert solvent such as benzene, toluene, or tetrahydrofuran in the presence of a catalytic amount of an organic acid such as p-toluenesulfonic acid, oxalic acid, adipic acid, or the like.

Reaction of the ketones (XXXV) with a hydroxylamine or hydrazine gives the corresponding oxime or hydrazone derivative (XXXVII). The reaction is carried out in a solvent mixture of pyridine in ethanol at a temperature of 50 °C to the reflux temperature of the solvent mixture. The hydrazone (XXXVII) where $R_7 = H$ may be acylated using an acyl halide or an acyl anhydride in the presence of an organic base such as pyridine or triethylamine in an organic solvent such as tetrahydrofuran or DMF. A catalyst such as 4-dimethylaminopyridine may be used.

Scheme 6:

a)

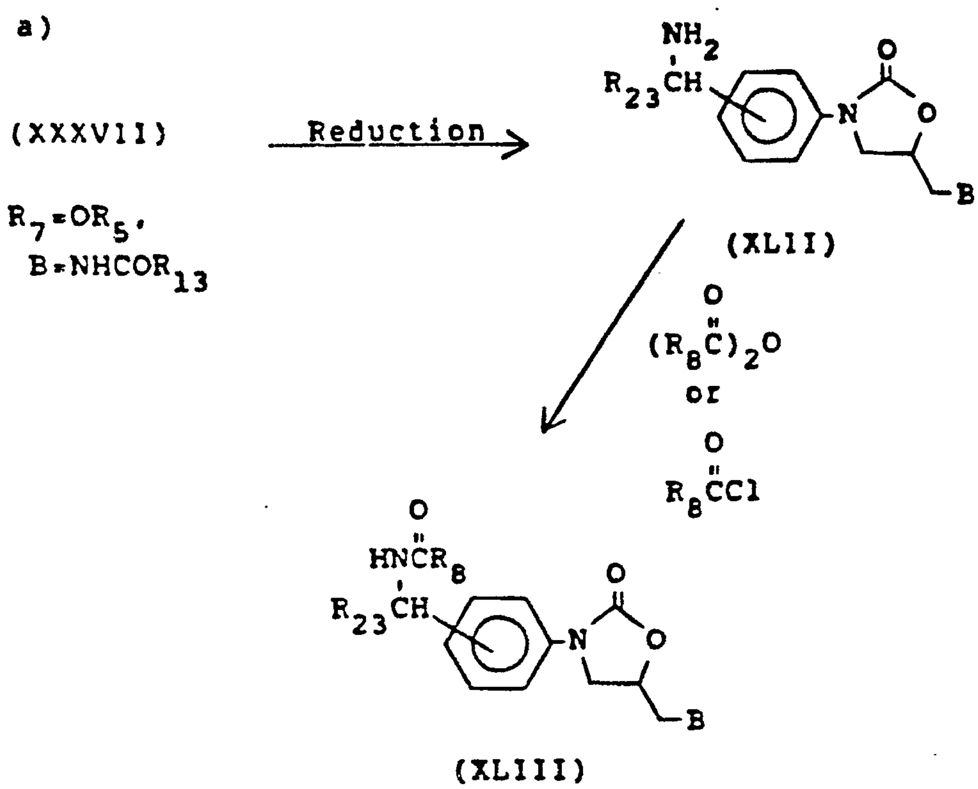


b)

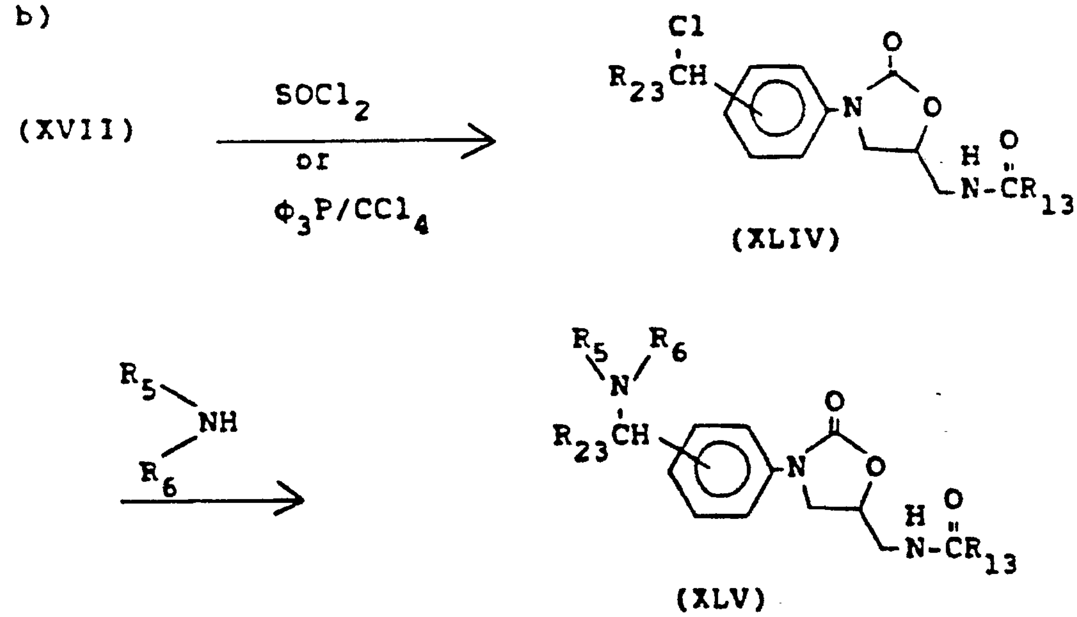


Amines (XLII) can be prepared by reduction as shown in Scheme 6 from the oxime derivatives (XXXVII) using a reducing agent such as 10% palladium-on-carbon in a solvent such as acetic acid or an alcohol solvent such as ethanol. The amines (XLII) may be acylated using an acyl anhydride or acyl halide in the presence of an organic base such as triethylamine or pyridine in an organic solvent such as methylene chloride or tetrahydrofuran. A catalyst such as 4-dimethylaminopyridine may be used.

A method for preparing secondary or tertiary amines (XLV) is shown in Scheme 6, Path b) wherein the halo compound (XLIV) can be prepared from the alcohol by reaction with thionyl chloride. Alternatively, the alcohol can be reacted with triphenylphosphine in carbon tetrachloride or carbon tetrabromide. The halo compound (XLIV) can then be reacted with an amine. For low boiling amines, the reaction can be carried out under pressure. For higher boiling amines, a mixture of (XLIV) is stirred optionally in a polar aprotic

solvent or an alcoholic solvent at a temperature of 50-150 ° C.

An alternative synthesis of compounds of structure (V) is shown in Scheme 7.

Scheme 7:

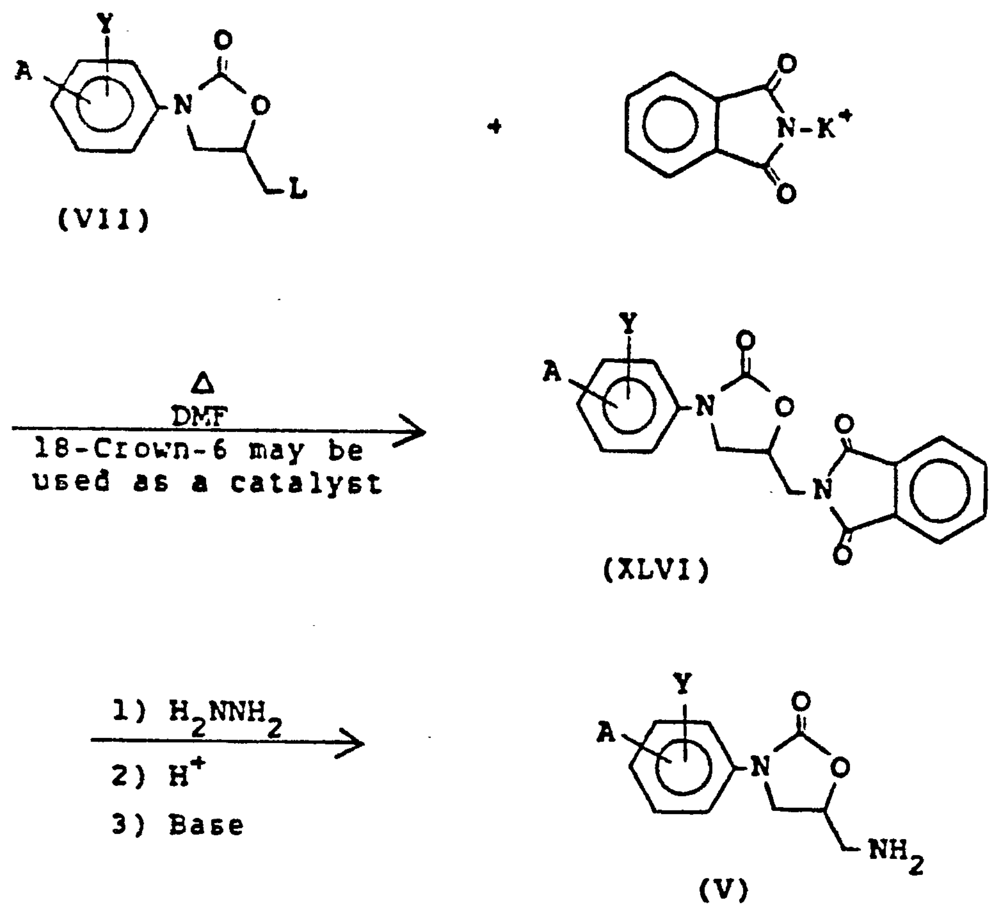


(VII) (XLVI) (V)

In Scheme 7, A may be H, or any of the groups previously shown except groups which are known to react with hydrazine. L may be any suitable leaving group such as I, Br, Cl, benzenesulfonyloxy, 4-toluenesulfonyloxy, methanesulfonyloxy, or trifluoromethanesulfonyloxy. The reaction is carried out by heating at temperatures of 25 ° to 150 ° C in a dipolar aprotic solvent such as DMF, DMAc, N-methylpyrrolidinone, tetramethylenesulfone or HMPA. The phthalimide group is then removed by treatment with hydrazine in alcohol at 20 ° C to 50 ° C for 5-30 hours followed by adjusting to neutral pH with acid. An alternate method is first to react (XLVI) with sodium sulfide, then to dehydrate with N,N-dicyclohexylcarbodiimide, followed by reaction with hydrazine and then treatment with dilute acid. This last method is very mild.

The alcohols (II) and halides (VII) required as starting materials are readily available by any of a number of standard methods for the preparation of oxazolidones. [M. E. Dyen and D. Swern, Chem. Rev,. 67, 197-246 (1967)].

Of these methods, the two which are noteworthy for the variety of compounds prepared are outlined in Scheme 8.

Scheme 8:

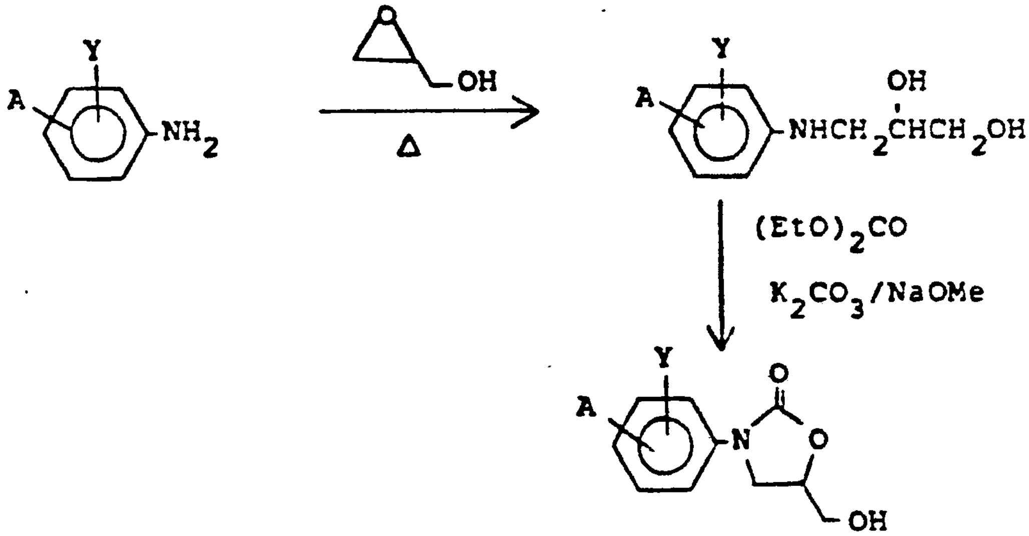


(II)

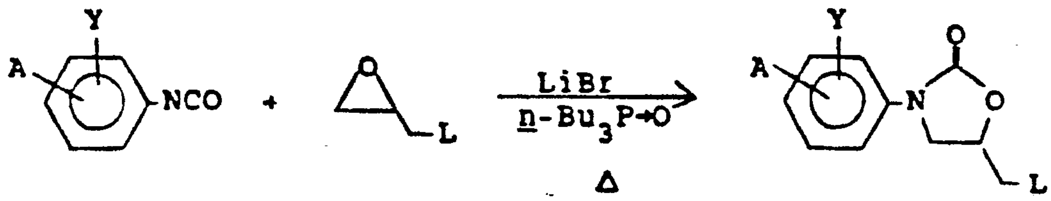


Pharmaceutically suitable salts of compounds of formula I can be prepared in a number of ways known in the art. Pharmaceutically suitable salts include those resulting from treatment with acetic, hydrochloric, sulfuric, phosphoric, succinic, fumaric, ascorbic, and glutaric acid.

Example 1

Preparation of (ℓ)-N-[3-[4-[1-(Acetylhydrazono)ethyl]phenyl]-2-oxo-5-oxazolidinylmethyl]acetamide (I;

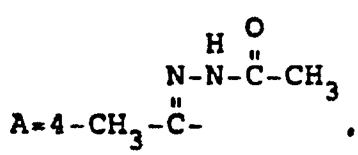

B = -$NHCOCH_3$)

Part A

Preparation of (ℓ)-5-hydroxymethyl-3-phenyl-2-oxazolidinone, 4-methylbenzenesulfonate, (I; A = H, B = $OSO_2C_6H_4Me$)

A mixture of 51.5 g of (ℓ)-5-hydroxymethyl-3-phenyl-2-oxazolidinone in 250 ml of dry pyridine was stirred under $N_2$ in an ice-bath as a solution of 53.0 g of p-toluenesulfonyl chloride in 50 ml of pyridine was added. After the addition, cooling was ceased, the mixture allowed to stand for one hour, and then a few drops of water were added (the temperature rose to 39 °C as the water reacted with the excess p-toluenesulfonyl chloride). The reaction mixture was poured into ice water; the white solid was filtered, washed well with water, and dried. The yield of product was 70.0 g, m.p. 146.3-147.8 °C. This product was used without further purification.

Part B

Preparation of (ℓ)-5-Azidomethyl-3-phenyl-2-oxazolidinone (I; A = H, B = $N_3$)

A mixture of 5.0 g (14.4 mmole) of (ℓ)-5-hydroxymethyl-3-phenyl-2-oxazolidinone, 4-methylbenzenesulfonate, 2.1 g sodium azide and 1 g 18-crown-6 in 35 ml of DMF was heated at 100° C for three hours. The mixture was poured into ice-water and filtered. The dried yield was 2.47 g, m.p. 71.5-72.5° C. This was recrystallized from diethyl ether to give 1.44 g of product, m.p. 72.5-73° C.

Part C

Preparation of (ℓ)-5-Aminomethyl-3-phenyl-2-oxazolidinone (I; A = H, B = $NH_2$)

A mixture of 37.0 (170 mmole) of (ℓ)-5-azidomethyl-3-phenyl-2-oxazolidinone, 26 ml of triethylamine, 19.5 ml of 1,3-propanedithiol in 150 ml of methanol was warmed to 50° C. Nitrogen was evolved (at the end of 2 hours, 3.9 liters had been measured). The solvent was removed and the residue crystallized on stirring with ether (crude yield, 28.3 g). This material was used without further purification.

Part D

Preparation of (ℓ)-N-(3-Phenyl-2-oxooxazolidin-5-ylmethyl)acetamide (I; A = H, B = $NHCOCH_3$)

A solution of 12.5 g (65.0 mmole) of (ℓ)-5-aminomethyl-3-phenyl-2-oxazolidinone in 50 ml of dry pyridine was stirred as 7 ml of acetic anhydride was added. The mixture was allowed to stand overnight, then concentrated. The residue was stirred with water and the solid filtered and dried; yield 10.2 g, m.p. 122.4-124.5° C. This was recrystallized from ethanol to give 5.02 g, m.p. 126.8-127.3° C. A second crop was obtained and recrystallized from ethanol to give 3.08 g, m.p. 127.3-127.8° C.

Part E

Preparation of (ℓ)-N-[3-(4-Acetylphenyl)-2-oxo-5-oxazolidinylmethyl)acetamide (I;

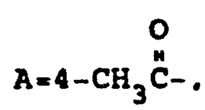

B = $NHCOCH_3$)

A 25 ml portion of methanesulfonic acid was stirred in a dry nitrogen atmosphere as 3.5 g (15 mmole) of (ℓ)-N-(3-phenyl-2-oxooxazolidin-5-ylmethyl)acetamide was added. To this was added 3 ml of acetic anhydride. The mixture was stirred for 2.5 hours, poured onto ice and the product was extracted with dichloromethane. The combined dichloromethane extracts were dried over sodium sulfate. The evaporation of the extract yielded a crude yellow product (4.6 g) which was recrystallized from acetonitrile to give 2.33 g of the title compound, m.p. 190.5-191.0° C.

Part F

Preparation of (ℓ)-N-[3-[4-(1-Hydrazonoethyl)phenyl]-2-oxo-5-oxazolidinylmethyl]acetamide(I;

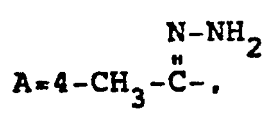

B = $-NHCOCH_3$)

A mixture of 1.7 g (6.2 mmole) of (ℓ)-N-[3-(4-acetylphenyl)-2-oxooxazolidin-5-ylmethyl]acetamide and

0.25 ml of 98% hydrazine in 25 ml of absolute ethanol was refluxed 1.5 hours and the reaction was about half way complete. An additional 0.125 ml of 98% hydrazine was added and the reaction was refluxed an additional 2 hours. The mixture was concentrated to dryness, the residue diluted with ethanol and the product crystallized. The yield was 1.2 g, m.p. >235 °C. The structure of this product was confirmed by NMR.

Part G

A solution of 1.0 g (3.62 mmole) of (ℓ)-N-[3-[4-(1-hydrazonoethyl)phenyl]-2-oxo-5-oxazolidinylmethyl]-acetamide in 10 ml of tetrahydrofuran and 10 ml of triethylamine was stirred as 0.5 ml of acetic anhydride was added. To this solution 0.1 g of 4-dimethylaminopyridine is added followed by 1 ml of acetic anhydride. After stirring 1 hour the mixture was concentrated and diluted with water and the solid was filtered; yield 1.01 g, m.p. 216.2-216.8 °C.

Example 2

Preparation of (dℓ)-N-[3-(4-Chlorophenyl)-2-oxooxazolidin-5-ylmethyl]carbamic acid, methyl ester (I; A = 4-Cl, B = $NHCO_2CH_3$)

Part A

Preparation of (dℓ)-5-(Bromomethyl)-3-(4-chlorophenyl)-2-oxazolidinone (I: A = 4-Cl, B = $NHCH_2Br$)

A hot solution of 2.0 g (23.0 mmole) of lithium bromide (LiBr) and 5.0 g (22.9 mmole) of tributylphosphine oxide in 800 ml xylenes (from which any water present had been azeotropically removed) was stirred under nitrogen as a solution of 50 g (0.33 mole) of p-chlorophenyl isocyanate and 27.9 ml (44.67 g. 0.326 mole) of epibromohydrin in 50 ml xylenes was added in a rapid manner dropwise. The reaction was cooled to room temperature and the solvent removed. The crude product was recrystallized from methanol to give 79 g of (dℓ)-5-(bromomethyl)-3-(4-chloro)phenyl-2-oxazolidinone.

Part B

Preparation of (dℓ)-2-Azido-N-[3-(4-chlorophenyl]-2-oxooxazolidin-5-ylmethyl]acetamide, (I; A=4-Cl, B = $NHCOCH_2N_3$)

A mixture of 20.0 g (68.8 mmole) of the above prepared compound, 4.70 g (72.3 mmole) sodium azide ($NaN_3$), and 1.52 g (6.88 mmole) 15-crown-5 in 100 ml dimethylformamide was heated with stirring under nitrogen to 85 °C and left overnight. The reaction was cooled to room temperature, poured into 400 ml ice water and the desired product was collected by filtration, washed with water and used moist in the subsequent reaction without further purification.

Part C

To a solution of the above compound in 1.5 liter absolute ethanol under nitrogen, 4.0 ml (4.20 g, 69.9 mmole) of glacial acetic acid was added followed by the addition of 1.0 g 10% palladium on charcoal (Pd/C) with stirring. Hydrogen was bubbled through the reaction mixture. After 1 hour, thin layer chromatography (TLC) showed no starting material. The reaction mixture was filtered through Celite®, washed with ethanol, and the solvent removed to give 18.0 g of (dℓ)-N-[2-oxo-3-(4-chlorophenyl)oxazolidin-5-yl]-methyl ammonium acetate.

The pH of a solution of 5.0 g (17.4 mmole) of the above compound in 100 ml THF/water (1:1) under nitrogen was adjusted to 10-11 with 25% NaOH solution and 2.7 ml (3.29 g, 34.8 mmole) of methyl chloroformate was added. After 1 hour, no starting material was observed by TLC and the reaction mixture was evaporated to give a brown solid that was washed with water. The crude product was purified via flash chromatography on silica gel (eluting with a gradient of methylene chloride/methanol) giving 1.89 g of the title compound, m.p. 123-124 °C, $M^+$ = 284.

Example 3

Preparation of (ℓ)-N-[3-(4-Bromophenyl)-2-oxooxazolidin-5-ylmethyl]acetamide, (I; A = 4-Br, B = $NHCOCH_3$)

Part A

Preparation of (ℓ)-5-hydroxymethyl-3-phenyl-2-oxazolidinone; 4-methylbenzenesulfonate, (I; A = H, B = $OSO_2C_6H_4Me$).

A solution of 149.0 g (0.77 mole) of (ℓ)-5-hydroxymethyl-3-phenyl-2-oxazolidinone in 500 ml of pyridine was stirred under nitrogen at room temperature, and a solution of 150.0g (0.79 mole) of tosyl chloride in 250 ml pyridine was added dropwise. After the addition, stirring was continued for 1 hour and the reaction mixture was allowed to stand at ambient temperature for 3 days. Tosyl chloride (30.0 g) was added and the reaction mixture was stirred for 4 hours followed by the addition of 18.9 g of tosyl chloride which was followed by a final addition of 50.0 g of tosyl chloride 2 hours later. After stirring overnight, the reaction mixture was cooled to 10° C and water was added in portions to decompose the excess tosyl chloride while maintaining the temperature below 20° C. The reaction mixture was diluted with 2 liters of water with cooling in an ice bath, and the crystalline product was collected by filtration and dried overnight in a vacuum oven at 60° C. The resulting solidified brown oil was triturated with 1 liter of acetonitrile to give an off-white solid; the mother liquor was concentrated followed by dilution with 3 liters of water to give more off-white solid. The combined solids totaling 199.0 g, were recrystallized from acetonitrile (decolorizing with charcoal). Repeated recrystallizations from acetonitrile failed to remove all impurities (m.p. 142.5-147.5° C and 145-152° C).

Part B

Preparation of (ℓ)-N-(3-Phenyl-2-oxooxazolidin-5-ylmethyl)phthalimide. (I; A = H,

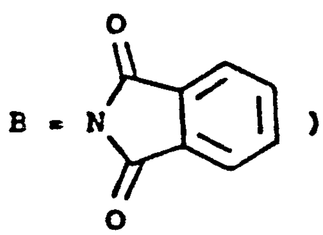

A mixture of 46.4 g (0.134 mole) of (ℓ)-5-hydroxymethyl-3-phenyl-2-oxazolidinone, methylbenzenesulfonate, 26.1 g (0.141 mole) potassium phthalimide, and 0.27 g (0.001 mole) 18-crown-6 in 200 ml freshly distilled dimethylformamide (DMF) was heated to 70° C with stirring under nitrogen. The resulting solution was allowed to stir overnight at 70° C, cooled to room temperature, and diluted to 1 liter with water. A white solid, (ℓ)-N-(3-phenyl-2-oxooxazolidin-5-ylmethyl) phthalimide, was collected by filtration, washed with water, and air dried to give 39.9 g, m.p. 167.3-168° C.

Part C

Preparation of (ℓ)-N-[3-(4-Bromophenyl)-2-oxooxazolidin-5-ylmethyl] phthalimide. (I; A = 4-Br,

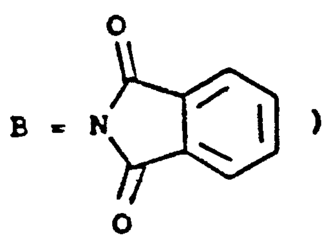

To a solution of 5.0 g (15.5 mmole) of (ℓ)-N-(3-phenyl-2-oxooxazolidin-5-ylmethyl) phthalimide in 75 ml

trifluoroacetic acid (TFA), a solution of 2.49 g (0.79 ml, 15.5 mmole) of bromine in 25 ml TFA was added dropwise with stirring at room temperature. After 4.5 hours, sodium bisulfite ($NaHSO_3$) was added, the reaction was filtered and the solvents removed. The recovered pale orange material was triturated with 200 ml of water to give 5.59 g of a white solid. Recrystallization of the crude product with acetonitrile gave 3.71 g of (ℓ)-N-[3-(4-bromophenyl)-2-oxooxazolidin-5-ylmethyl] phthalimide. m.p. 190-191 °C.

Part D

Preparation of (ℓ)-5-Aminomethyl-3-(4-bromophenyl)-2-oxazolidinone, (I: A = 4-Br, B = $NH_2$)

To a suspension of 5.85 g (14.6 mmole) of (ℓ)-N-[3-(4-bromophenyl)-2-oxooxazolidin-5-ylmethyl] phthalimide in 50 ml absolute ethanol, 0.51 ml (0.515 g, 16.1 mmole) of hydrazine was added with stirring. The reaction mixture was allowed to stir overnight, filtered, washed with ethanol, and the solvent removed. The resulting oil was suspended in 100 ml of water, the pH adjusted to 3 with hydrochloric acid, and allowed to stir overnight. The pH (now 7) of the reaction mixture was readjusted back to 3 with hydrochloric acid, stirred for 1 hour, filtered and the solvents removed. The resulting white solid was suspended in ethyl ether, filtered, washed with ethyl ether and air dried to give 1.76g of the product as the hydrochloride salt.

Part E

The pH of a solution of 840 mg (2.7 mmole) of (ℓ)-5-aminomethyl-3-(-4-bromophenyl)-2-oxazolidinone hydrochloride salt in 50 ml of tetrahydrofuran/water (2:1) cooled in an ice bath under nitrogen, was adjusted to 10 with 25% sodium hydroxide (NaOH) solution followed by the addition of 0.52 ml (0.55 g, 5.5 mmole) of acetic anhydride. The reaction was stirred with cooling for 1 hour, warmed to room temperature and stirred overnight. The solvents were removed and the crude product was triturated with water, filtered, washed with water, and air dried to provide 400 mg of (ℓ)-N-[3-(4-bromophenyl)-2-oxooxazolidin-5-ylmethyl)acetamide, m.p. 179.8-182.1 °C, $M^+$ = 312, 314.

Example 4

Preparation of (dℓ)-N-[3-(4-Chlorophenyl)-2-oxooxazolidin-5-ylmethyl)acetamide (I; A = 4-Cl, B = $NHCOCH_3$)

The pH of a solution of 5.0 g (17.4 mmole) of (dℓ)-N-[3-(4-chlorophenyl)-2-oxooxazolidin-5-yl]methyl) ammonium acetate in 100 ml tetrahydrofuran/water (1:1) was adjusted to 10-11 with 25% sodium hydroxide solution and the solution was placed under nitrogen. With stirring, 3.3 ml (3.56 g, 34.9 mmole) of acetic anhydride was added. After 1 hour, thin layer chromatography showed no starting material and the solvents were evaporated. The crude product was washed with water and dried to give 3.87 g of the title compound, m.p. 155-156 °C, $M^+$ = 268.

Example 5

Preparation of (dℓ)-N-[3-(4-Fluorophenyl)-2-oxooxazolidin-5-ylmethyl]acetamide (I; A = 4-F, B = $NHCOCH_3$)

Substitution of p-fluorophenyl isocyanate for the p-chlorophenyl isocyanate in the procedure of Example 2 gave 2g of (dℓ)-N-[3-(4-fluorophenyl)-2-oxooxazolidin-5-ylmethyl]acetamide, m.p. 135-136 °C, $M^+$ = 252.

Example 6

Preparation of (ℓ)-N-[3-[4-(1-Hydroxyethyl)phenyl]-2-oxo-5-oxazolidinylmethyl]acetamide (I;

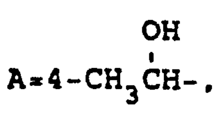

B = $-NHCOCH_3$)

A 2.00 g (7.2 mmole) portion of (ℓ)-N-[3-(4-acetylphenyl)-2-oxooxazolidin-5-ylmethyl]acetamide in 50 ml of ethanol was stirred as a solution of 1.0 g of sodium borohydride in 5 ml of water was added. The solid went into solution and at the end of 15 minutes thin layer chromatography showed the reaction had gone to completion. The solvent was removed by concentration under vacuum, the residue diluted with water and made acidic with dilute HCl and the product extracted into dichloromethane. The extracts were dried over sodium sulfate and concentrated. The residue was recrystallized from acetonitrile to give 0.66 g, m.p. 128.8-129.8 °C.

Examples 7-9

Using the procedures of Example 1 and Example 6, the following compounds were or can be prepared.

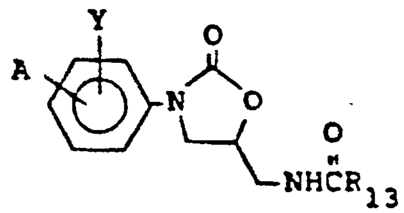

Table 1

| Ex. | A,Y | $R_{13}$ | m.p. °C | Isomer |
|---|---|---|---|---|
| 7 | 4-$ClCH_2CO$, H | $CH_3$ | 175.8-178.8 | ℓ |
| 8 | OH<br>4-$CH_3CH_2CH$, H | $OCH_3$ | | ℓ |
| 9 | 4-$CF_3CH_2CO$, H | $CH_3$ | | ℓ |

Example 10

Preparation of (ℓ)-N-[3-(4-Iodophenyl)-2-oxo-5-oxazolidinylmethyl]acetamide (I; A = 4-I, B = $NHCOCH_3$)

A solution of 23.5 g (0.10 mole) of (ℓ)-N-(3-phenyl-2-oxooxazolidin-5-ylmethyl)acetamide and 44.2 g of silver trifluoroacetate in 200 ml of chloroform was stirred at ambient temperature as a solution of 27.9 g (0.11 mole) of iodine in 200 ml of chloroform was added. The solid silver trifluoroacetate became coated with a gum. After 4 hours, 20.0 g of silver trifluoroacetate was added and stirring continued an additional 2 hours. The mixture was filtered and the solid was washed with chloroform and dichloromethane. The chloroform solution was then washed with aqueous solution of sodium carbonate. The dried chloroform solution was concentrated to give 10.0 g of crude product, m.p. 147-170 °C. This was recrystallized from acetonitrile to give 8.0 g of the title compound, m.p. 194-195 °C.

Example 11

Preparation of (ℓ)-N-[3-(4-Ethynylphenyl)-2-oxo-5-oxa zolidinylmethyl]acetamide (I; A = 4-HC≡C, B = $NHCOCH_3$)

Part A

Preparation of (ℓ)-N-[3-(4-Trimethylsilylethynylphenyl)-2-oxo-5-oxazolidinylmethyl]acetamide (I; A = 4-$(CH_3)_3SiC≡C$, B = $NHCOCH_3$)

To 5.0 g of (ℓ)-[3-(4-iodophenyl)-2-oxo-5-oxazolidinylmethyl]acetamide and 1.6 g of trimethylsilylacetylene in 20 ml of dimethylformamide and 20 ml of triethylamine was added 0.193 g of bis-(triphenylphosphine)palladium (II) chloride and 0.26 g of copper (I) iodide. After stirring for 4.5 hours at 45° C, the reaction solution was concentrated. The residue was dissolved in acetonitrile and ethyl ether and washed with water. The acetonitrile-ethyl ether solution was concentrated to dryness and purified via chromatography on silica gel (eluent: ethylene glycol dimethyl ether-cyclohexane [1:1]) to give 3.4 g of the title compound, m.p. 145-145° C. The mass spectrum of this sample gave a molecular ion peak of 330.

Part B

To a 2.0 g sample of (ℓ)-N-[3-(4-trimethylsilylethynylphenyl)-2-oxo-5-oxazolidinylmethyl]acetamide dissolved in approximately 50 ml of methanol was added 10 ml of 1 N potassium hydroxide at ambient temperature. After stirring for 90 minutes the reaction solution was acidified to a pH of 3 with dilute aqueous hydrochloric acid. Water was added to the acidified solution followed by extraction with dichloromethane. The dried dichloromethane extracts were concentrated to crude solids. The solids were purified by chromatography on silica gel (eluent: ethylene glycol dimethyl ether - hexane [1:1]). The pure fractions collected were concentrated and then recrystallized from dichloromethane and hexane to yield 0.98 g of the title compound, m.p. 169.5-171.5° C. The mass spectrum of a sample prepared in a similar fashion gave a molecular ion peak of 258.

Dosage Forms

The antibacterial agents of this invention can be administered by any means that produces contact of the active agent with the agent's site of action in the body of a mammal. They can be administered by any conventional means available for use in conjunction with pharmaceuticals, either as individual therapeutic agents or in a combination of therapeutic agents. They can be administered alone, but are generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health, and weight of the recipient; nature and extent of symptoms; kind of concurrent treatment; frequency of treatment; and the effect desired. Usually a daily dosage of active ingredient can be about 5 to 20 milligrams per kilogram of body weight. Ordinarily, when the more potent compounds of this invention are used, 5 to 15, and preferably 5 to 7.5 milligrams per kilogram per day, given in divided doses 2 to 4 times a day or in sustained release form, is effective to obtain desired results. These drugs may also be administered parenterally.

Projected therapeutic levels in humans should be attained by the oral administration of 5-20 mg/kg of body weight given in divided doses two to four times daily. The dosages may be increased in severe or life-threatening infections.

Dosage forms (compositions) suitable for internal administration contain from about 1.0 milligram to about 500 milligrams of active ingredient per unit. In these pharmaceutical compositions the active ingredient will ordinarily be present in an amount of about 0.5 - 95% by weight based on the total weight of the composition.

The active ingredient can be administered orally in solid dosage forms, such as capsules, tablets, and powders, or in liquid dosage forms, such as elixirs, syrups, and suspensions, it can also be administered parenterally, in sterile liquid dosage forms.

Gelatin capsules contain the active ingredient and powdered carriers, such as lactose, sucrose, mannitol, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents, and if necessary, buffer substances. Antioxidants such as sodium bisulfate,

sodium sulfite, or ascorbic acid either alone or combined are suitable stabilizing agents. Also used are citric acid and its salts and sodium EDTA. In addition parenteral solutions can contain preservatives, such as benzalkonium chloride, methyl-or propyl-paraben, and chlorobutanol.

Suitable pharmaceutical carriers are described in Remington's Pharmaceutical Sciences, A. Osol, a standard reference text in this field.

Useful pharmaceutical dosage forms for administration of the compounds of this invention can be illustrated as follows:

Capsules

A large number of unit capsules are prepared by filling standard two-piece hard gelatin capsules each with 75 milligrams of powdered active ingredient, 150 milligrams of lactose, 24 milligrams of talc, and 6 milligrams of magnesium stearate.

Soft Gelatin Capsules

A mixture of active ingredient in soybean oil is prepared and injected by means of a positive displacement pump into gelatin to form soft gelatin capsules containing 75 milligrams of the active ingredient. The capsules are washed and dried.

Tablets

A large number of tablets are prepared by conventional procedures so that the dosage unit is 75 milligrams of active ingredient, 0.2 milligrams of colloidal silicon dioxide, 5 milligrams of magnesium stearate, 250 milligrams of microcrystalline cellulose, 11 milligrams of cornstarch and 98.8 milligrams of lactose. Appropriate coatings may be applied to increase palatability or delay absorption.

Injectable

A parenteral composition suitable for administration by injection is prepared by stirring 1.5% by weight of active ingredient in 10% by volume propylene glycol and water. The solution is made isotonic with sodium chloride and sterilized.

Suspension

An aqueous suspension is prepared for oral administration so that each 5 milliliters contain 75 milligrams of finely divided active ingredient, 200 milligrams of sodium carboxymethyl cellulose, 5 milligrams of sodium benzoate, 1.0 grams of sorbitol solution, U.S.P., and 0.025 milliliters of vanillin.

Utility

Test results indicate that the novel compounds of this invention are biologically active against gram negative and gram positive bacteria including betalactamase producing Staphylococcus aureus isolates. These agents are potentially useful for the treatment of both human and animal bacterial infections including diseases of the respiratory, gastrointestinal, genito-urinary and central nervous systems; blood; interstitial fluids; soft tissue; and bone.

As shown in Table 2, compounds of formula I exert an in vitro antibacterial effect. A standard microdilution method (Conrath, Theodore B., 1972 Handbook of Microtiter Procedures, Dynatech Corporation, Cambridge, Massachusetts) with Mueller-Hinton broth is used to determine the 24-hour minimal inhibitory concentrations (MIC's) for test strains of Staphylococcus epidermidis and Escherichia coli.

The in vivo potency of these compounds is exemplified by the data summarized in Tables 3 and 4. Determinations of in vivo efficacy are performed by inoculating mice intraperitoneally with cultures of the infecting organism diluted to produce 90-100% mortality in control animals within twenty-four hours. The diluents used were trypticase soy broth for E. coli and 5% aqueous hog gastric mucin for Staphylococcus aureus infections. The compounds are dissolved or suspended in 0.25% aqueous Methocel® (Methocel®: Hydroxypropyl Methylcellulose E15 Premium, Dow Chemical Company) for oral administration or sterile distilled water containing 5% dimethylsulfoxide (Fisher Scientific Company, Fairlawn, N.J.) for subcutaneous administration. The mice are dosed at the time of infection and again at four hours post-infection. Mortality

is recorded daily until test termination seven days post infection and the 50 percent effective dose, $ED_{50}$, is calculated by the Reed-Muench method (Reed, L. G. and Muench, H., "A simple method of estimating fifty percent end points," American Journal of Hygiene, 27, 493-497 (1938).

## *IN VITRO* BROTH DILUTION
## MINIMAL INHIBITORY CONCENTRATIONS

Microdilution Broth MIC in µg/ml

| Ex. No. | *Staphylococcus epidermidis* | *Escherichia coli* |
|---|---|---|
| 1 | 6.2 | >200.0 |
| 2 | >128.0 | >128.0 |
| 3 | 6.25 | >200.0 |
| 4 | 128.0 | >128.0 |
| 5 | >128.0 | >128.0 |
| 6 | 3.1 | 50.0 |
| 7 | 1.6 | 100.0 |
| 10 | 12.5 | >200.0 |
| 11 | 16.0 | >128.0 |

## Table 3

**IN VIVO EFFICACY OF ORALLY ADMINISTERED COMPOUNDS IN MOUSE INTRAPERITONEAL INFECTIONS**

| | Infecting Bacterial Organism | |
|---|---|---|
| Ex. No. | Staphylococcus aureus | Escherichia coli |
| | $ED_{50}$ (mg/kg) | $ED_{50}$ (mg/kg) |
| 1 | < 4.4 | 24.7 |
| 2 | 79.2 | >120.0 |
| 3 | 6.9 | >40.0 |
| 4 | 42.0 | >120.0 |
| 5 | 23.6 | >120.0 |
| 6 | 2.1 | 17.4 |
| 7 | <4.4 | .100.0 |
| 10 | 50.0 | N.T. |

[1] $ED_{50}$ = 50 percent effective dose in mg/kg

[2] N.T. = Not tested.

## Table 4

**IN VIVO EFFICACY OF COMPOUNDS ADMINISTERED SUBCUTANEOUSLY IN MOUSE INTRAPERITONEAL INFECTIONS**

| Ex. No. | Infecting Bacterial Organism: Staphylococcus aureus $ED_{50}$ (mg/kg) | Escherichia coli $ED_{50}$ (mg/kg) |
|---|---|---|
| 1 | 6.9 | >120.0 |
| 2 | 80.5 | N.T. |
| 3 | 4.7 | >40.0 |
| 4 | 10.5 | >120.0 |
| 5 | 21.9 | >120.0 |
| 6 | <1.3 | 26.3 |
| 7 | <4.4 | 100.0 |
| 10 | 12.2 | >120.0 |

[1] $ED_{50}$ = 50 percent effective dose in mg/kg
[2] N.T. = Not tested.

## Claims

**Claims for the following Contracting States: BE, CH, DE, FR, GB, IT, LI, LU, NL, SE**

**1.** A compound of the formula

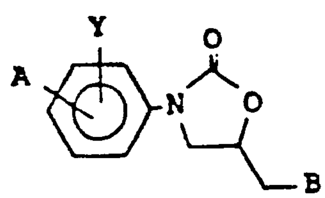

(I)

wherein, for the ℓ, and mixtures of the d and ℓ stereoisomers of the compound.

A is halogen, alkynyl of 2-5 carbon atoms, $-COR_{23a}$,

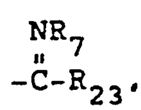, 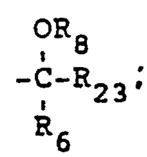;

$R_5$ and $R_6$ are alkyl of 1-4 carbon atoms;

$R_7$ is

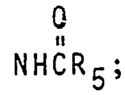

$R_8$ is H;
$R_{23}$ is H;
$R_{23a}$ is alkyl of 1-4 carbon atoms substituted with one or more halogens;
B is

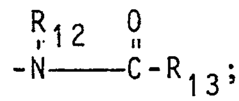

$R_{12}$ is H;
$R_{13}$ is $C_1$-$C_4$ alkyl, $-OR_{18}$;
$R_{18}$ is $C_1$-$C_4$ alkyl;
or a pharmaceutically suitable salt thereof;
provided that: when A is F, then B is not $NHCO_2CH_3$ and when B is $-NH-CO-CH_3$, then A and Y taken together cannot be $O-CH_2-O$.

2. A compound of Claim 1 with the stereochemical formula

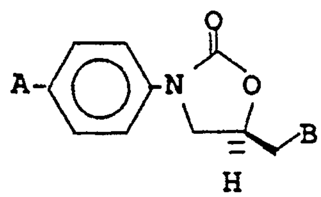

wherein A, substituted in the para position, is

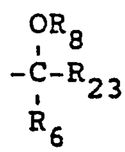

3. A compound of Claim 1 with the stereochemical formula

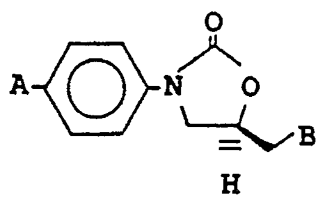

wherein
B is

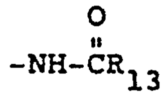 ;

$R_{13}$ is $CH_3$, $OR_{18}$;
$R_{18}$ is alkyl of 1-4 carbon atoms.

4. A compound of Claim 3 wherein A, substituted in the para position, is

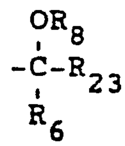

5. The compound of Claim 1 which is (l)-N-[3-[4-(1-hydroxyethyl)phenyl]-2-oxo-5-oxazolidinylmethyl]-acetamide.

6. A pharmaceutical composition comprising a suitable pharmaceutical carrier and at least one compound of claims 1 - 5.

7. A process for preparing compounds of Claims 1 - 5 characterized by:
(a) contacting a compound of the formula

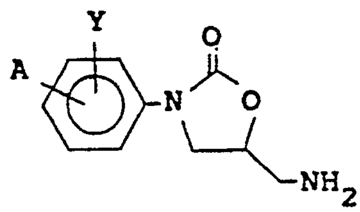

where A is hydrogen, bromine, chlorine or fluòrine with an acid chloride, anhydride or sulfonyl chloride or with a carboxylic acid in the presence of an appropriate coupling reagent to prepare a compound of the formula

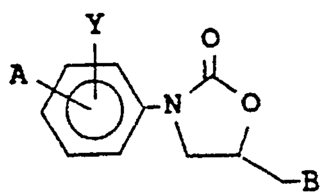

where B is

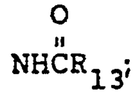;

(b) contacting the compound of step (a) where A is hydrogen with
(i) an anhydride such as acetic anhydride in the presence of an organic acid such as methanesulfonic acid or with an appropriate carboxylic acid in the presence of a coupling reagent such as methanesulfonic anhydride to prepare a compound of the formula $R_{23}$ or

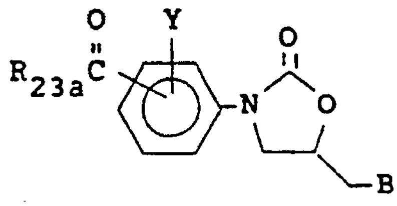

where B is

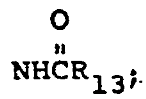

(ii) silver trifluoroacetate and iodine to form an iodo-compound which can be isolated per se or reacted by palladium coupling with an appropriate alkyne moiety to prepare a compound of formula (I) where A is alkynyl;

(c) contacting an acylated compound of step (b) with hydrazine followed by acylation with an acyl halide or an acyl anhydride in the presence of an organic base such as pyridine or triethylamine or a catalytic amount of a base such as 4-dimethylaminopyridine to prepare a compound of formula (I) wherein

A is

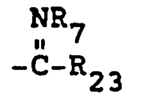

**Claims for the following contracting state: AT**

**1.** A process for preparing a compound of the formula

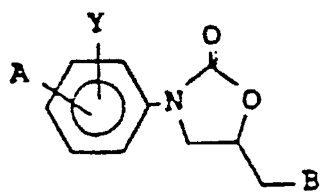

(I)

wherein, for the ℓ, and mixtures of the d and ℓ stereoisomers of the compound.

A is halogen, alkynyl of 2-5 carbon atoms, $-COR_{23a}$,

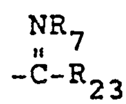,
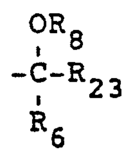;

$R_5$ and $R_6$ are alkyl of 1-4 carbon atoms;

$R_7$ is

$$NH\overset{O}{\overset{\|}{C}}R_5;$$

| | |
|---|---|
| $R_8$ | is H; |
| $R_{23}$ | is H; |
| $R_{23a}$ | is alkyl of 1-4 carbon atoms substituted with one or more halogens; |
| B | is |

$$-\overset{R_{12}}{\overset{|}{N}}-\overset{O}{\overset{\|}{C}}-R_{13};$$

| | |
|---|---|
| $R_{12}$ | is H; |
| $R_{13}$ | is $C_1$-$C_4$ alkyl, $-OR_{18}$; |
| $R_{18}$ | is $C_1$-$C_4$ alkyl; |

or a pharmaceutically suitable salt thereof;
provided that: when A is F, then B is not $NHCO_2CH_3$ and when B is $-NH-CO-CH_3$, then A and Y taken together cannot be $O-CH_2-O$, which comprises

(a) contacting a compound of the formula

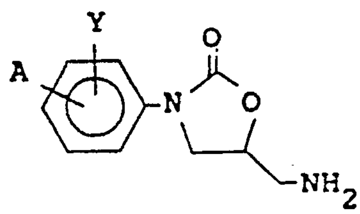

where A is hydrogen, bromine, chlorine or fluorine with an acid chloride, anhydride or sulfonyl chloride or with a carboxylic acid in the presence of an appropriate coupling reagent to prepare a compound of the formula

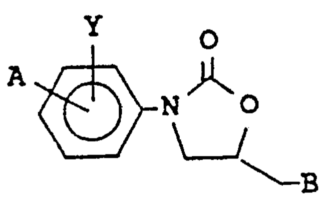

where B is

$$NH\overset{O}{\overset{\|}{C}}R_{13};$$

(b) contacting the compound of step (a) where A is hydrogen with

(i) an anhydride such as acetic anhydride in the presence of an organic acid such as methanesulfonic acid or with an appropriate carboxylic acid in the presence of a coupling reagent such as methanesulfonic anhydride to prepare a compound of the formula
$R_{23}$ or

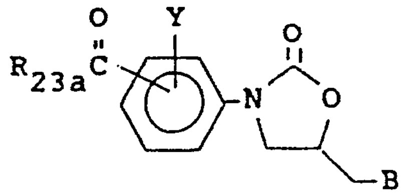

where B is

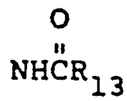;

(ii) silver trifluoroacetate and iodine to form an iodo-compound which can be isolated per se or reacted by palladium coupling with an appropriate alkyne moiety to prepare a compound of formula (I) where A is alkynyl;

(c) contacting an acylated compound of step (b) with hydrazine followed by acylation with an acyl halide or an acyl anhydride in the presence of an organic base such as pyridine or triethylamine or a catalytic amount of a base such as 4-dimethylaminopyridine to prepare a compound of formula (I) wherein A is

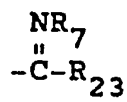 .

2. A process of Claim 1 for preparing a compound with the stereochemical formula

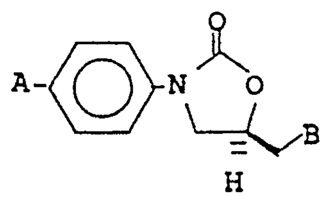

wherein A, substituted in the para position, is

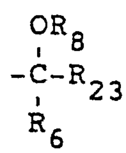 .

3. A process of Claim 1 for preparing a compound with the stereochemical formula

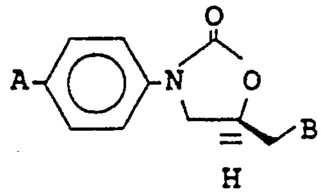

wherein

B is

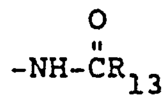 ;

$R_{13}$ is $CH_3$, $OR_{18}$;

$R_{18}$ is alkyl of 1-4 carbon atoms.

**4.** A process of Claim 3 wherein A, substituted in the para position, is

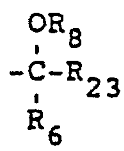 .

**5.** A process of Claim 1 wherein the compound prepared is (ℓ)-N-[3-[4-(1-hydroxyethyl)phenyl]-2-oxo-5-oxazolidinylmethyl]-acetamide.

**6.** A process for preparing a pharmaceutical composition comprising combining a suitable pharmaceutical carrier and at least one compound prepared according to Claims 1 - 5.

**Revendications**

**Revendications pour l'Etat contractant suivant: AT**

**1.** Un procédé de préparation d'un composé de formule:

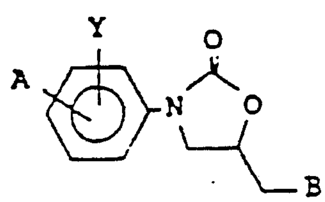

(I)

dans laquelle, pour les stéréo-isomères ℓ et les mélanges des stéréo-isomères d et ℓ du composé,

A est un halogène, un radical alkynyle ayant 2 à 5 atomes de carbone,

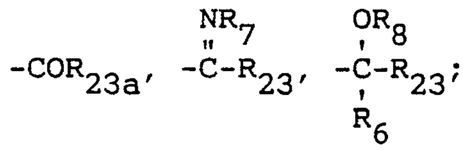

$R_5$ et $R_6$ représentent chacun un radical alkyle ayant 1 à 4 atomes de carbone;

$R_7$ est

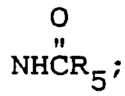

$R_8$ est H;

$R_{23}$ est H;

$R_{23a}$ est un radical alkyle ayant 1 à 4 atomes de carbone, substitué par un ou plusieurs atomes d'halogène;

B est

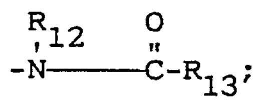

$R_{12}$ est H;

$R_{13}$ est un radical alkyle en $C_1$-$C_4$, ou -$OR_{18}$; $R_{18}$ est un radical alkyle en $C_1$-$C_4$;

ou un de ses sels pharmaceutiquement acceptables;

à la condition que, quant A est F, B ne soit pas -$NHCO_2CH_3$, et que, quand B est -NH-CO-$CH_3$, A et Y, pris ensemble, ne puissent être O-$CH_2$-O,

lequel procédé consiste:

(a) à mettre en contact un composé de formule:

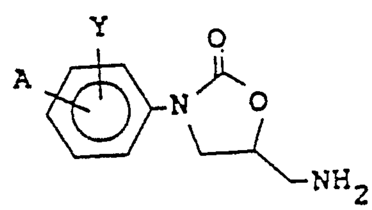

dans laquelle A est un atome d'hydrogène, de brome, de chlore ou de fluor, avec un chlorure d'acide, un anhydride d'acide ou du chlorure de sulfonyle ou avec un acide carboxylique en présence d'un réactif de copulation approprié, pour préparer un composé de formule:

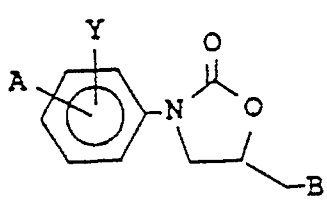

dans laquelle B est

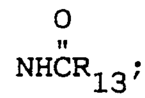

(b) à mettre en contact le composé de l'étape (a) où A est un atome d'hydrogène, avec:

(i) un anhydride, tel que l'anhydride acétique, en présence d'un acide organique tel que l'acide méthanesulfonique, ou avec un acide carboxylique approprié, en présence d'un réactif de copulation tel que l'anhydride méthanesulfonique, pour préparer un composé de formule $R_{23}$ or

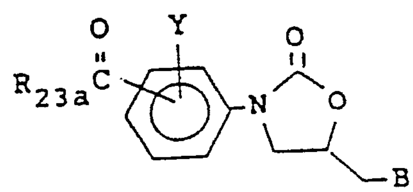

ou B est

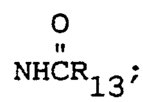

(ii) du trifluoracétate d'argent et de l'iode pour former un composé iodé pouvant être isolé en tant que tel ou pouvant être mis à réagir, par couplage au palladium avec un fragment alkyle approprié pour préparer un composé de formule (I) dans laquelle A est un radical alkynyle;

(c) à mettre en contact un composé acylé de l'étape (b) avec de l'hydrazine, opération suivie d'une acylation avec un halogénure d'acyle ou un anhydride d'acyle en présence d'une base organique telle que la pyridine ou la triéthylamine ou d'une quantité catalytique d'une base telle que la 4-diméthylaminopyridine pour préparer un composé de formule (I) dans laquelle A est

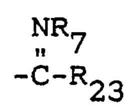

**2.** Procédé selon la revendication 1, pour préparer un composé ayant la formule stéréochimique suivante:

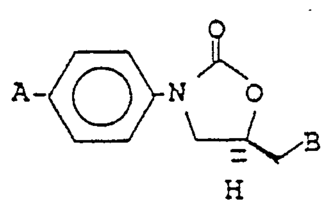

dans laquelle A, substitué en position para, est:

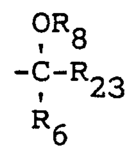

3. Un procédé selon la revendication 1, pour préparer un composé ayant la formule stéréochimique suivante:

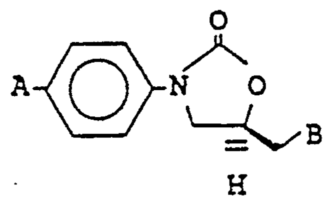

dans laquelle

B est

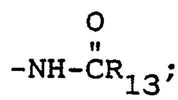

$R_{13}$ est $CH_3$, $OR_{18}$;

$R_{18}$ est un radical alkyle ayant 1 à 4 atomes de carbone.

4. Un procédé selon la revendication 3, dans lequel A, substitué en position para est:

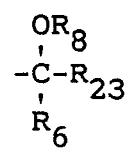

5. Un procédé selon la revendication 1, dans lequel le composé préparé est le (ℓ)-N-{3-{4-(1-hydroxyéthyl)phényl}-2-oxo-5-oxazolidinylméthyl}-acétamide.

6. Un procédé de préparation d'une composition pharmaceutique, qui consiste à combiner un excipient pharmaceutique approprié et au moins un composé préparé selon les revendications 1 à 5.

**Revendications pour les Etats contractants suivants: BE, CH, DE, FR, GB, IT, LI, LU, NL, SE**

1. Un composé de formule:

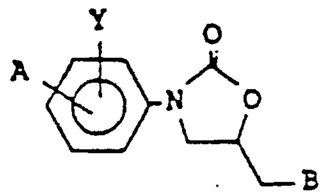

(I)

dans laquelle, pour les stéréo-isomères ℓ et les mélanges des stéréo-isomères d et ℓ du composé,

A est un halogène, un radical alkynyle ayant 2 à 5 atomes de carbone, $-COR_{23a}$,

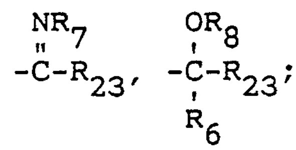

$R_5$ et $R_6$ représentent chacun un radical alkyle ayant 1 à 4 atomes de carbone;

$R_7$ est

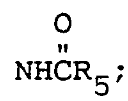

$R_8$ est H;

$R_{23}$ est H;

$R_{23a}$ est un radical alkyle ayant 1 à 4 atomes de carbone, substitué par un ou plusieurs atomes d'halogène;

B est

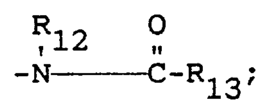

$R_{12}$ est H;

$R_{13}$ est un radical alkyle en $C_1$-$C_4$, ou $-OR_{18}$;

$R_{18}$ est un radical alkyle en $C_1$-$C_4$;

ou un de ses sels pharmaceutiquement acceptables;

à la condition que, quant A est F, B ne soit pas $-NHCO_2CH_3$, et que, quand B est $-NH-CO-CH_3$, A et Y, pris ensemble, ne puissent être $O-CH_2-O$.

**2.** Un composé selon la revendication 1, ayant la formule stéréochimique suivante:

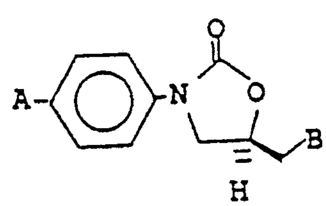

dans laquelle A, substitué en position para, est:

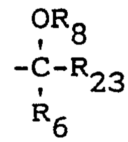

3. Un composé selon la revendication 1, ayant la formule stéréochimique suivante:

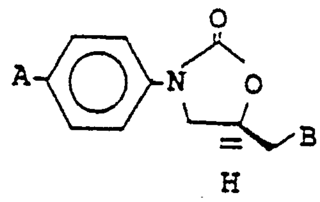

dans laquelle

B est

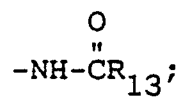

$R_{13}$ est $CH_3$, $OR_{18}$;

$R_{18}$ est un radical alkyle ayant 1 à 4 atomes de carbone.

4. Un composé selon la revendication 3, dans lequel A, substitué en position para est

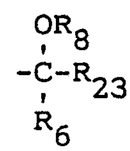

5. Le composé selon la revendication 1, qui est le (ℓ)-N-{3-{4-(1-hydroxyéthyl)phényl}-2-oxo-5-oxazolidinylméthyl}-acétamide.

6. Une composition pharmaceutique comprenant un excipient pharmaceutique acceptable et au moins un composé selon les revendications 1 à 5.

7. Un procédé de préparation des composés selon les revendications 1 à 5 caractérisé en ce qu'il consiste:

(a) à mettre en contact un composé de formule:

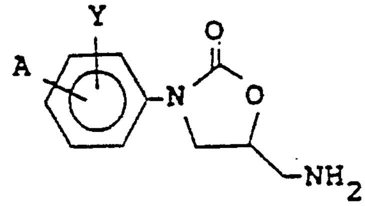

dans laquelle A est un atome d'hydrogène, de brome, de chlore ou de fluor, avec un chlorure d'acide, un anhydride d'acide ou du chlorure de sulfonyle ou avec un acide carboxylique en présence d'un réactif de copulation approprié, pour préparer un composé de formule:

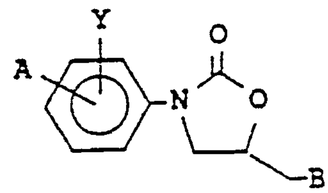

dans laquelle B est

$$NH\overset{O}{\overset{\|}{C}}R_{13};$$

(b) à mettre en contact le composé de l'étape (a) où A est un atome d'hydrogène, avec:

(i) un anhydride, tel que l'anhydride acétique, en présence d'un acide organique tel que l'acide méthanesulfonique, ou avec un acide carboxylique approprié, en présence d'un réactif de copulation tel que l'anhydride méthanesulfonique, pour préparer un composé de formule $R_{23}$ or

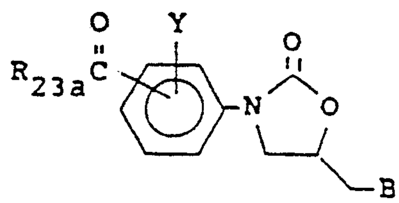

où B est

$$NH\overset{O}{\overset{\|}{C}}R_{13};$$

(ii) du trifluoracétate d'argent et de l'iode pour former un composé iodé pouvant être isolé en tant que tel ou pouvant être mis à réagir, par couplage au palladium avec un fragment alkyle approprié pour préparer un composé de formule (I) dans laquelle A est un radical alkynyle;

(c) à mettre en contact un composé acylé de l'étape (b) avec de l'hydrazine, opération suivie d'une acylation avec un halogénure d'acyle ou un anhydride d'acyle en présence d'une base organique telle que la pyridine ou la triéthylamine ou d'une quantité catalytique d'une base telle que la 4-diméthylaminopyridine pour préparer un composé de formule (I) dans laquelle A est

$$-\overset{NR_7}{\overset{\|}{C}}-R_{23}$$

**Patentansprüche**

**Patentansprüche für folgende Vertragsstaaten: BE, CH, DE, FR, GB, IT, LI, LU, NL, SE**

**1.** Verbindung der Formel

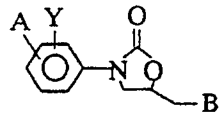,

(I)

in der für die ℓ- und Gemische der d- und ℓ-Stereoisomeren der Verbindung

A Halogen, Alkinyl mit 2 bis 5 Kohlenstoff-Atomen, $-COR_{23a}$,

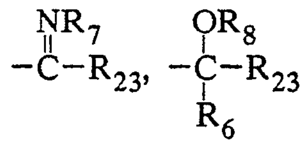

ist;

$R_5$ und $R_6$ Alkyl mit 1 bis 4 Kohlenstoff-Atomen sind;

$R_7$

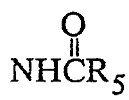

ist;

$R_8$ H ist;

$R_{23}$ H ist;

$R_{23a}$ Alkyl mit 1 bis 4 Kohlenstoff-Atomen ist, das mit einem oder mehreren Halogen-Atomen substituiert ist;

B

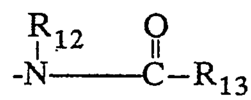

ist;

$R_{12}$ H ist;

$R_{13}$ $C_1$-$C_4$-Alkyl, $-OR_{18}$ ist;

$R_{18}$ $C_1$-$C_4$-Alkyl ist,

oder ein pharmazeutisch geeignetes Salz derselben,
mit der Maßgabe, daß wenn A F ist, dann B nicht $NHCO_2CH_3$ ist und
wenn B $-NH-CO-CH_3$ ist, dann A und Y zusammen genommen nicht $O-CH_2-O$ sein können.

**2.** Verbindung nach Anspruch 1 mit der stereochemischen Formel

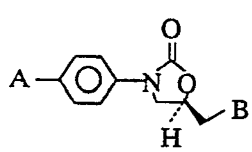,

worin
A, substituiert in der para-Stellung,

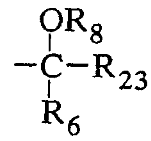

ist.

3. Verbindung nach Anspruch 1 mit der stereochemischen Formel

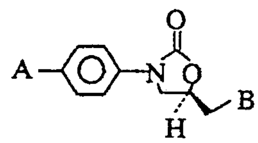

,

worin

B

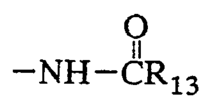

ist,

$R_{13}$ $CH_3$, $OR_{18}$ ist;

$R_{18}$ Alkyl mit 1 bis 4 Kohlenstoff-Atomen ist.

4. Verbindung nach Anspruch 3, worin A, substituiert in der para-Stellung,

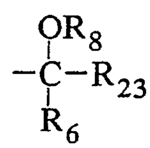

ist.

5. Verbindung nach Anspruch 1, die (ℓ)-N-[3-[4-(1-Hydroxyethyl)phenyl]-2-oxo-5-oxazolidinylmethyl]-acetamid ist.

6. Pharmazeutische Zusammensetzung, umfassend einen geeigneten pharmazeutischen Träger und wenigstens eine Verbindung nach den Ansprüchen 1 bis 5.

7. Verfahren zur Herstellung der Verbindungen nach den Ansprüchen 1 bis 5, umfassend
(a) das In-Berührung-Bringen einer Verbindung der Formel

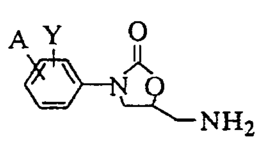

,

in der A Wasserstoff, Brom, Chlor oder Fluor ist, mit einem Säurechlorid, -anhydrid oder Sulfonylchlorid oder mit einer Carbonsäure in Gegenwart eines geeigneten Kupplungs-Reagens zur Herstellung einer Verbindung der Formel

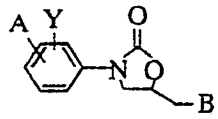

worin B

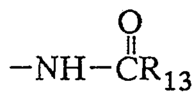

ist;

(b) das In-Berührung-Bringen der Verbindung aus Schritt (a), in der A Wasserstoff ist, mit

(i) einem Anhydrid wie Acetanhydrid in Gegenwart einer organischen Säure wie Methansulfonsäure oder mit einer geeigneten Carbonsäure in Gegenwart eines Kupplungs-Reagens wie Methansulfonsäureanhydrid zur Herstellung einer Verbindung der Formel

$R_{23}$ oder

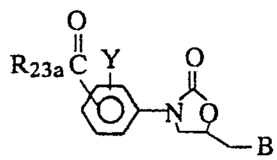

worin B

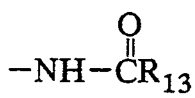

ist;

(ii) Silbertrifluoracetat und Iod zur Bildung einer Iod-Verbindung, die an sich isoliert werden oder durch Palladium-Kupplung mit einer geeigneten Alkin-Struktureinheit umgesetzt werden kann, um eine Verbindung herzustellen, in der A Alkinyl ist;

(c) das In-Berührung-Bringen einer acylierten Verbindung aus Schritt (b) mit Hydrazin und anschließende Acylierung mit einem Acylhalogenid oder einem Acylanhydrid in Gegenwart einer organischen Base wie Pyridin oder Triethylamin oder einer katalytischen Menge einer Base wie 4-Dimethylaminopyridin, um eine Verbindung der Formel (I) herzustellen, in der

A

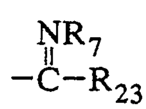

ist.

**Patentansprüche für folgenden Vertragsstaat: AT**

1. Verfahren zur Herstellung einer Verbindung der Formel

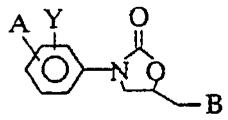

,

(I)

in der für die ℓ- und Gemische der d- und ℓ-Stereoisomeren der Verbindung

A Halogen, Alkinyl mit 2 bis 5 Kohlenstoff-Atomen, $-COR_{23a}$,

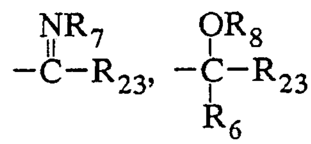

ist;

$R_5$ und $R_6$ Alkyl mit 1 bis 4 Kohlenstoff-Atomen sind;

R 7

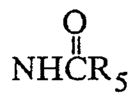

ist;

$R_8$ H ist;

$R_{23}$ H ist;

$R_{23a}$ Alkyl mit 1 bis 4 Kohlenstoff-Atomen ist, das mit einem oder mehreren Halogen-Atomen substituiert ist;

B

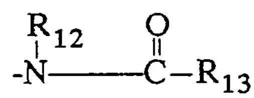

ist;

$R_{12}$ H ist;

$R_{13}$ $C_1$-$C_4$-Alkyl, $-OR_{18}$ ist;

$R_{18}$ $C_1$-$C_4$-Alkyl ist,

oder eines pharmazeutisch geeigneten Salzes derselben,
mit der Maßgabe, daß wenn A F ist, dann B nicht $NHCO_2CH_3$ ist und
wenn B $-NH-CO-CH_3$ ist, dann A und Y zusammen genommen nicht $O-CH_2-O$ sein können,
umfassend

(a) das In-Berührung-Bringen einer Verbindung der Formel

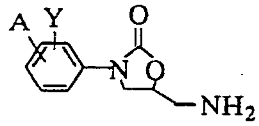

,

in der A Wasserstoff, Brom, Chlor oder Fluor ist, mit einem Säurechlorid, -anhydrid oder Sulfonylchlorid oder mit einer Carbonsäure in Gegenwart eines geeigneten Kupplungs-Reagens zur Herstellung einer Verbindung der Formel

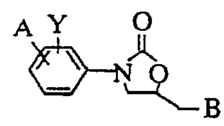

worin B

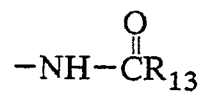

ist;

(b) das In-Berührung-Bringen der Verbindung aus Schritt (a), in der A Wasserstoff ist, mit

(i) einem Anhydrid wie Acetanhydrid in Gegenwart einer organischen Säure wie Methansulfonsäure oder mit einer geeigneten Carbonsäure in Gegenwart eines Kupplungs-Reagens wie Methansulfonsäureanhydrid zur Herstellung einer Verbindung der Formel $R_{23}$ oder

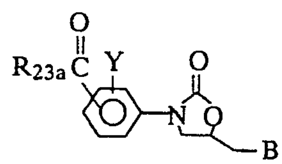

worin B

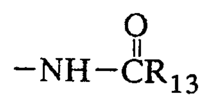

ist;

(ii) Silbertrifluoracetat und Iod zur Bildung einer Iod-Verbindung, die an sich isoliert werden oder durch Palladium-Kupplung mit einer geeigneten Alkin-Struktureinheit umgesetzt werden kann, um eine Verbindung herzustellen, in der A Alkinyl ist;

(c) das In-Berührung-Bringen einer acylierten Verbindung aus Schritt (b mit Hydrazin und anschließende Acylierung mit einem Acylhalogenid oder einem Acylanhydrid in Gegenwart einer organischen Base wie Pyridin oder Triethylamin oder einer katalytischen Menge einer Base wie 4-Dimethylaminopyridin, um eine Verbindung der Formel (I) herzustellen, in der A

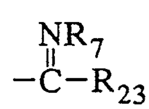

ist.

**2.** Verfahren nach Anspruch 1 zur Herstellung einer Verbindung mit der stereochemischen Formel

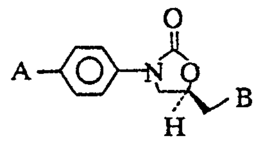

,

worin
A, substituiert in der para-Stellung,

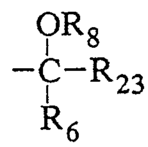

ist.

3. Verfahren nach Anspruch 1 zur Herstellung einer Verbindung mit der stereochemischen Formel

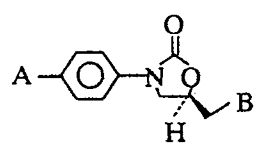

,

worin
B

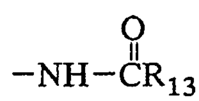

ist, $R_{13}$ $CH_3$, $OR_{18}$ ist;
$R_{18}$ Alkyl mit 1 bis 4 Kohlenstoff-Atomen ist.

4. Verfahren nach Anspruch 3, worin A, substituiert in der para-Stellung,

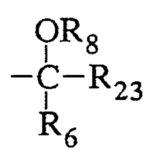

ist.

5. Verfahren nach Anspruch 1, worin die Verbindung (ℓ)-N-[3-[4-(1-Hydroxyethyl)phenyl]-2-oxo-5-oxazolidinylmethyl]acetamid ist.

6. Verfahren zur Herstellung einer pharmazeutischen Zusammensetzung, umfassend das Kombinieren eines geeigneten pharmazeutischen Trägers und wenigstens einer Verbindung, die nach einem der Ansprüche 1 bis 5 hergestellt ist.